# EUROPEAN PATENT SPECIFICATION

(11) **EP 3 938 339 B1**
(45) Date of publication and mention of the grant of the patent: **29.04.2026**
(21) Application number: 20768886.2
(22) Date of filing: 13.03.2020
(51) Int. Cl.: C07C 51/48, A23D 9/02, C07C 51/42, C07C 57/03, C11B 3/16, C11B 3/06, C11B 1/02, C11B 3/00

(54) **METHODS OF OBTAINING LIPIDS FROM A MICROBIAL CELL COMPOSITION**
VERFAHREN ZUR GEWINNUNG VON LIPIDEN AUS EINER MIKROBIELLEN ZELLZUSAMMENSETZUNG
PROCÉDÉS D'OBTENTION DE LIPIDES À PARTIR D'UNE COMPOSITION DE CELLULES MICROBIENNES

(30) Priority: 14.03.2019 US 201962818500 P; 15.03.2019 US 201962818915 P
(43) Date of publication of application: 19.01.2022
(73) Proprietor: DSM IP Assets B.V., 6221 BE Maastricht (NL)
(72) Inventor: ADUGNA, Negash, Columbia, Maryland 21045 (US)
(74) Representative: Hoyng Rokh Monegier B.V.
(86) International application number: PCT/US2020/022528
(87) International publication number: WO 2020/186128

(56) References cited:
- WO-A1-2015/095688
- WO-A1-2015/095696
- WO-A1-2018/013670
- US-A1- 2015 218 484
- US-A1- 2016 318 840
- US-A1- 2016 319 217

## Description

This application is an international application claiming priority to US Provisional Application No's 62/818500, filed 14 March 2019 and US Provisional Application 62/818915, filed 15 March 2019.

### FIELD OF THE INVENTION

The present invention relates to processes for obtaining a lipid from a composition comprising microbial cells by concentrating a population of microbial cells, lysing the population of microbial cells, demulsifying the lysed cells, and recovering the lipid.

### BACKGROUND OF THE INVENTION

A typical process for obtaining lipids from a microbial cell, such as polyunsaturated fatty acids, involves growing microorganisms that are capable of producing the desired lipid in a fermenter, pond or bioreactor, separating the fermentation broth comprising a microbial cell biomass, drying the microbial cell biomass, and separating the lipids by solvent extraction. Steps in the separation can include diluting a fermentation broth with water, centrifuging the diluted broth, lysing the microbial cells, and extracting an intracellular lipid from the lysed cells by adding a water-immiscible solvent to the mixture in which the lipid is soluble, e.g., hexane.

Another method of extraction to remove a lipid from a microbial cell is lysing a cell in a fermentation broth using mechanical force (e.g., homogenization), enzymatic treatment, or chemical treatment to disrupt the cell walls. Lipid can be extracted from the resulting composition comprising lipids, microbial cell biomass, and water using an organic solvent, e.g., isopropyl alcohol. The lipid can be separated mechanically from the composition and the alcohol must be removed from both the lipid and the aqueous biomass waste stream. See, e.g., International Pub. Nos. WO 01/76385 and WO 01/76715.

However, industrial scale production of lipids using either of the above processes requires a large amount of volatile and flammable organic solvent, thereby creating hazardous operating conditions. The use of organic solvents in the extraction process can also necessitate using an explosion-proof lipid recovery system, thereby adding to the cost of lipid recovery. Moreover, use of an organic solvent in extracting lipid from a microbial cell can generate an organic solvent waste stream that requires a complete solvent recovery system or a proper method of disposal, which further increases the overall production cost of lipid extraction. For example, strict limits on volatile organic compound (VOC) emissions require greater manpower and added cost to vessels and other equipment.

Therefore, there is a need for a process for obtaining lipids from a cell which does not use an organic solvent in order to minimize the problems mentioned above. Several processes have been proposed for separating a lipid from a cell without the use of an organic solvent. For example, U.S. Patent No. 6,750,048 discloses an aqueous washing process whereby an emulsion is washed with aqueous washing solutions until a substantially non-emulsified lipid is obtained. However, in some embodiments, this process requires multiple washing steps, which require substantial cost and time. U.S. Patent No. 7,431,952 discloses a process whereby lysed cells are centrifuged to remove cell wall debris and then oils are extracted and purified. However, this process provides a crude oil that requires extensive further purification. Document US2016/319217 discloses a process for obtaining lipids from a microbial cell involving pasteurizing and lysing the cell to obtain a lysed cell composition, further subjected to a step of demulsification wherein the demulsification is carried out by either raising or lowering the pH of the lysed cell composition.

### BRIEF SUMMARY OF THE INVENTION

The present invention is directed to a process for obtaining a lipid from a composition comprising microbial cells as set out in the appended set of claims.

The present disclosure is also directed to the following embodiments, which are not according to the invention and are present for illustrative purposes only. A first embodiment relates to a lipid obtained by any of the processes of the present invention.

In some embodiments, the lipid has an overall aroma intensity of 3 or less. In some embodiments, the lipid has an overall aromatic intensity of 2 or less.

In some embodiments, the lipid comprises at least 10% by weight of a desired polyunsaturated fatty acid (PUFA). In some embodiments, the lipid comprises at least 10% by weight of docosahexaenoic acid ("DHA"), at least 5% by weight of docosapentaenoic acid n-3 ("DPA n-3"), at least 10% by weight of eicosapentaenoic acid ("EPA"), and/or at least 10% by weight of ARA.

In some embodiments, the lipid comprises at least 20% by weight eicosapentaenoic acid and less than 5% by weight each of arachidonic acid, docosapentaenoic acid n-6, oleic acid, linoleic acid, linolenic acid, eicosaenoic acid, erucic acid, and stearidonic acid.

In some embodiments, the lipid has an anisidine value of 26 or less.

In some embodiments, the lipid is a crude lipid. In some embodiments, the crude lipid optionally has less than 5% by weight or volume of an organic solvent. In some embodiments, the crude lipid has no organic solvent.

Another embodiment which is not according to the invention, and which is present for illustration purposes only is a lipid having an anisidine value of 26 or less, a peroxide value of 5 or less, a phosphorus content of 100 ppm or less, and optionally less than 5% by weight or volume of an organic solvent.

A further embodiment which is not according to the invention, and which is present for illustration purposes only is an extracted lipid comprising a triglyceride fraction of at least 70% by weight, wherein the docosahexaenoic acid content of the triglyceride fraction is at least 40% by weight, wherein the docosapentaenoic acid n-6 content of the triglyceride fraction is from at least 0.5% by weight to 6% by weight, and wherein the oil has an anisidine value of 26 or less. In some embodiments, the extracted lipid has a ratio of docosahexaenoic acid to docosapentaenoic acid n-6 that is greater than 6:1.

The processes of the present invention provide a particular advantage over processes known in the art by improving the amount and/or quality of the extracted lipid composition, decreasing the processing time for one or more steps in the process for obtaining the lipid from a cell composition, decreasing the amount of reagents necessary for obtaining the lipid from a cell composition, reducing the amount of hazardous chemicals utilized in the process as well as the amount of hazardous waste generated by the process, increasing the yield of RBDW (refined, bleached, deodorized, winterized) oil, reducing variability in commercial scale production, and reducing the overall monetary costs for extracting the lipid.

### BRIEF DESCRIPTION OF THE DRAWINGS/FIGURES

The accompanying drawings, which are incorporated herein and form a part of the specification, illustrate the present invention and, together with the description, further serve to explain the principles of the invention and to enable a person skilled in the pertinent art to make and use the invention.

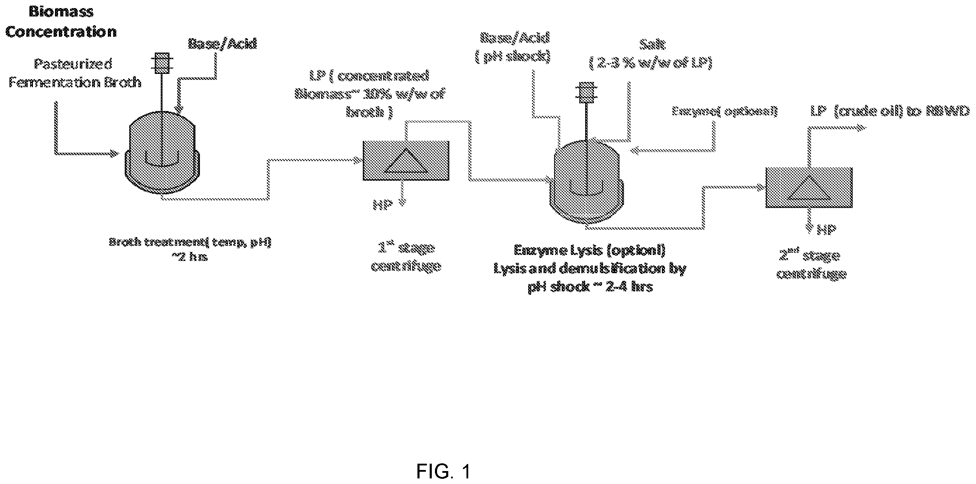

FIG. 1 provides a schematic flow chart describing processes of the present invention.

### DETAILED DESCRIPTION OF THE INVENTION

Described herein is a process for obtaining a lipid from a composition comprising microbial cells, the process comprising: treating the composition comprising the microbial cells by heating the composition to a temperature of about 60°C to about 95°C at a pH of about 9 to about 11 under agitation for a period of time from about 1 hour to about 6 hours; separating the treated microbial cells into a light phase consisting essentially of a concentrated microbial cell composition and a heavy phase consisting essentially of fermentation medium and aqueous material; sequentially, in one or more cycles, raising the pH of the concentrated microbial cell composition to 10 or above for a period of time, followed by lowering the pH of the concentrated microbial cell composition to a pH of less than 6 for a period of time to lyse the concentrated microbial cell composition, wherein the raising and lowering of the pH also demulsifies the lysed cell composition ("pH shock"), optionally in the presence of salt; separating a lipid from the demulsified cell composition, and recovering the lipid.

According to the present invention, the process comprises:
(a) treating a composition comprising the microbial cells by heating the composition to a temperature of about 60°C to about 95°C at a pH of about 9 to about 11 under agitation for a period of time from about 1 hour to about 6 hours;
(b) separating the treated microbial cells into a light phase consisting essentially of concentrated microbial cells and a heavy phase consisting essentially of fermentation medium and aqueous material;
(c) adjusting the pH of the concentrated microbial cell composition of the light phase to a pH of about 10 to about 12 by adding a base and maintaining the pH of about 10 to about 12 for at least 1 hour;
(d) adjusting the pH of the composition of (c) to a pH of about 3 to about 6 by adding an acid and maintaining the pH of about 3 to about 6 for at least 0.5 hours;
(e) optionally repeating steps (c) and (d) until the composition is sufficiently demulsified;
(f) separating the lipid from the demulsified lysed cell composition; and
(g) recovering the lipid.

In some embodiments, the composition comprising microbial cells is heated during the concentration step (a) to a temperature of about 60°C to about 95°C at a pH of about 9 to about 11 under agitation for a period of time from about 1 hour to about 6 hours, about 1 hour to about 5 hours, about 1 hour to about 4 hours, about 1 hour to about 3 hours, about 2 hours to about 6 hours, about 3 hours to about 6 hours, about 4 hours to about 6 hours, or about 2 hours to about 4 hours or longer. During this step, the surface chemistry of the cells is disrupted, thus allowing the cells to concentrate which can facilitate subsequent separation.

In various embodiments, the cell composition of (a) is cooled to a temperature of about 70-80°C and separated into a light phase consisting essentially of concentrated microbial cells and a heavy phase consisting essentially of fermentation medium and aqueous material by centrifuging at a temperature of about 0°C to about 100°C at 4000 *x* g to 11000 *x* g for 10 seconds to 1 minute. In various embodiments, the cell composition of (a) is cooled to a temperature of about 70-80°C and separated into a light and a heavy phase by centrifuging at about 4000 *x* g to about 11000 *x* g for 10 seconds to 3 minutes.

In some embodiments, the concentrated microbial cell composition of the light phase is reduced to about 5 to about 25% by weight of the fermentation broth, significantly reducing the overall volume of the composition without significantly reducing the microbial cell population.

Following concentration, the light phase is lysed and demulsified using one or more (e.g., 2, 3, 4, 5, 6, or more) pH shock cycles. In some embodiments, the pH of the light phase is first adjusted to 10 or above by the addition of a base during the lysis and demulsification process for a period of time of about 5 minutes, about 10 minutes, about 20 minutes, about 30 minutes, about 50 minutes, about one hour, about 1.5 hours, about 2 hours, about 2.5 hours, about 3 hours or longer. The pH is next adjusted to 6 or below by addition of an acid during the lysis and demulsification process for a period of time of about 5 minutes, about 10 minutes, about 20 minutes, about 30 minutes, about 50 minutes, about one hour, about 1.5 hours, about 2 hours, about 2.5 hours, about 3 hours or longer. In some embodiments, the process comprises heating the lysed cell composition prior to addition of a base for 5 minutes to 10 hours, 10 minutes to 4 hours, 30 minutes to 2 hours, 30 minutes to 1 hour, 30 minutes to 1.5 hours, 1 hour to 1.5 hours, or 1 hour to 2 hours.

In some embodiments, an enzyme is added to the concentrated cell composition of the light phase prior to the pH shock cycle(s). In this embodiment, the process comprises lysing the cell composition of the light phase by adjusting the pH of the concentrated cell composition to between about 7 to about 8.5, heating the cell composition to a temperature of about 60° to about 80°C for a period of time in the presence of an enzyme capable of disrupting the cell wall. In various embodiments, the temperature is about 60°C to about 65°C, about 65°C to about 70°C, about 70°C to about 75°C, about 75°C to about 80°C, about 80°C to about 85°C, about 85°C to about 90°C, about 60°C, about 65°C, about 70°C, about 75°C, about 80°C, about 85°C, or about 90°C for a period of time of 20 minutes, about 30 minutes, about 50 minutes, about one hour, about 1.5 hours, about 2 hours, about 2.5 hours, about 3 hours or longer.

In some embodiments, the lysis step does not involve an enzyme.

In some embodiments, the process comprises contacting the concentrated microbial cell composition or the lysed cell composition with a salt to facilitate the demulsification of the lysed cell composition. In some embodiments, the salt is selected from the group consisting of: alkali metal salts, alkali earth metal salts, sulfate salts and combinations thereof. In some embodiments, the salt is sodium chloride, potassium chloride, or sodium sulfate. The salt can be added prior to or during the pH shock cycle(s).

Following demulsification, the composition is again separated into light and heavy phases using, for example, centrifugation, membrane filtration, drum filtration, gravity settling, freeze drying, and the like. In one embodiment, the separation is performed using centrifugation at a temperature of 60°C to 85°C and a speed of 4000 RPM to 8000 RPM. Following separation, the light phase consists primarily of oil whereas the heavy phase consists primarily of fermentation medium and aqueous material.

In some embodiments, the process comprises, prior to the concentration step: washing, dilution centrifuging, evaporating, pasteurization, cooling or a combination thereof, a fermentation broth that includes the cell. In some embodiments, the process comprises concentrating the lysed cell composition.

The present invention is also directed to a process for obtaining a lipid, the process comprising refining a crude lipid of the present invention. In some embodiments, the refining is selected from the group consisting of: caustic refining, degumming, acid treatment, alkali treatment, cooling, heating, bleaching, deodorizing, deacidification, and combinations thereof.

Generally, the processes of the present invention do not utilize an organic solvent in order to extract or otherwise separate a lipid. Thus, in some embodiments, an organic solvent is not added to a fermentation broth comprising a microbial cell, is not added to a cell composition, is not added to a lysed cell composition, or is not added to a lipid during a process of the present invention in an amount or concentration sufficient to extract a lipid. As used herein, an "organic solvent" refers to a solvent that includes at least one carbon atom. As used herein, "solvent" refers to an agent that is hydrophobic or lipophilic and is not a lipid. As used herein, "hydrophobic" refers to an agent that is repelled from a mass of water. As used herein, "lipophilic" refers to an agent that dissolves lipids. Organic solvents that are not used in a process of the present invention include, but are not limited to, polar solvents, non-polar solvents, water-miscible solvents, water-immiscible solvents, and combinations thereof. Non-limiting examples of organic solvents include substituted and unsubstituted C₄-C₈ alkyls (e.g., hexane and the like), C₃-C₁₂ cycloalkyls, C₄-C₁₂ alkenes, C₁-C₈ alcohols (e.g., iso-propanol and the like), C₁-C₈ aldehydes, C₄-C₈ ethers, C₁-C₈ esters, C₆-C₁₂ aryls, C₁-C₈ amides, C₃-C₁₂ heteroaryls, and combinations thereof.

In some embodiments, an organic solvent can be added to a microbial cell composition, a lysed cell composition, or a demulsified cell composition. In such embodiments, the organic solvent is added in a concentration less than 5%, less than 4%, less than 3%, less than 2%, less than 1%, less than 0.5%, less than 0.1%, or less than 0.05% by volume. An organic solvent as defined herein can be optionally added to a lysed cell composition, for example, as a component of a base and/or a salt for contacting with the lysed cell composition. However, in such embodiments the organic solvent is present in a concentration such that the lipid is not substantially extracted from the microbial cell composition, lysed cell composition, or demulsified cell composition by the solvent (i.e., in a concentration of less than 5%, less than 4%, less than 3%, less than 2%, less than 1%, less than 0.5%, less than 0.1%, or less than 0.05% by volume or weight).

In some embodiments, the process of the present invention does not include washing, e.g., with water, or the process reduces the number of washings of, a lysed cell composition or a demulsified cell composition. "Washing" refers to a process of diluting a composition with, e.g., water or buffer and removing the water or buffer, e.g., by centrifugation. Washing a cell composition can decrease the overall yield of a lipid obtained from a cell. In the present invention, the washing can be decreased by 1 time, 2 times, 3 times or more.

### Definitions

As used herein, "concentrating" refers to removing water and/or fermentation media from a composition. Concentrating can include, but is not limited to, evaporating, chemical drying, centrifuging, membrane filtration, and the like, and combinations thereof.

As used herein, "lipid" or "oil" refers to one or more fatty acids (including free fatty acids and esters of fatty acids), phospholipids, triacylglycerols (i.e. triglycerides), diacylglycerides, monoacylglycerides, lysophospholipids, soaps, phosphatides, waxes, sterols and sterol esters, carotenoids, xanthophylls, hydrocarbons, and other lipids known to one of ordinary skill in the art. Lipids include polar lipids and neutral lipids.

As used herein, "polar lipid" refers to lipids that contain a polar group and are more readily soluble in polar solvents. Polar lipids include phospholipids. As used herein, "phospholipid" refers to lipids having a phosphate group. As used herein, "neutral lipid" refers to lipids that do not contain areas of polarity and are more readily soluble in non-polar solvents. Neutral lipids include triacylglycerols (TAG).

As used herein, the phases of separation following, for example, centrifugation are based on separation according to specific gravity. Following the initial treatment step but prior to demulsification, the "light phase" consists primarily of material having a lower specific gravity, e.g., the concentrated cell composition (or "biomass"). The "heavy phase" consists primarily of material having a higher specific gravity, e.g., the aqueous phase (e.g., water, salts, sugar). For the purposes of the present invention, the separated light phase may contain minimal residual aqueous material. Following demulsification and/or lysis, the light phase consists primarily of concentrated microbial cell composition, whereas the heavy phase consists primarily of fermentation medium and aqueous material. At this step, there is also a low amount of a third phase which is similar in composition to the heavy phase.

Fatty acids are classified based on the length and saturation characteristics of the carbon chain. Fatty acids are termed short chain, medium chain, or long chain fatty acids based on the number of carbons present in the chain. Fatty acids are termed saturated fatty acids when no double bonds are present between the carbon atoms, and are termed unsaturated fatty acids when double bonds are present. Unsaturated long chain fatty acids are monounsaturated when only one double bond is present and are polyunsaturated when more than one double bond is present.

Fatty acids present in the lipid can have 4 to 28 carbon atoms. In some embodiments, a lipid comprises one or more polyunsaturated fatty acids. Polyunsaturated fatty acids (PUFAs) are classified based on the position of the first double bond from the methyl end of the fatty acid: omega-3 (n-3) fatty acids contain a first double bond at the third carbon, while omega-6 (n-6) fatty acids contain a first double bond at the sixth carbon. For example, docosahexaenoic acid ("DHA") is an omega-3 long chain polyunsaturated fatty acid (LC-PUFA) with a chain length of 22 carbons and 6 double bonds, often designated as "22:6 n-3." For the purposes of this application, long chain polyunsaturated fatty acids (LC-PUFAs) are defined as fatty acids of 18 or more carbon chain length, and are preferably fatty acids of 20 or more carbon chain length, containing 3 or more double bonds. LC-PUFAs of the omega-6 series include, but are not limited to, di-homo-gamma-linoleic acid (C20:3n-6), arachidonic acid (C20:4n-6) ("ARA"), docosatetraenoic acid or adrenic acid (C22:4n-6), and docosapentaenoic acid (C22:5n-6) ("DPA n-6"). The LC-PUFAs of the omega-3 series include, but are not limited to, eicosatrienoic acid (C20:3n-3), eicosatetraenoic acid (C20:4n-3), eicosapentaenoic acid (C20:5n-3) ("EPA"), docosapentaenoic acid (C22:5n-3) ("DPA n-3"), and docosahexaenoic acid (C22:6n-3) ("DHA"). The LC-PUFAs also include fatty acids with greater than 22 carbons and 4 or more double bonds including, but not limited to, C24:6(n-3) and C28:8(n-3).

The terms "fatty acid," "polyunsaturated fatty acid," and "PUFA" include not only the free fatty acid form, but other forms as well, such as the triacylglycerol (TAG) form, the phospholipid (PL) form and other esterified forms. As used herein, the terms "ester" and "esterified" refer to the replacement of the hydrogen in the carboxylic acid group of a PUFA molecule with another substituent. Typical esters are known to those in the art, a discussion of which is provided by Higuchi, T. et al., Pro-drugs as Novel Delivery Systems, Vol. 14, A.C.S. Symposium Series, Bioreversible Carriers in Drug Design, Edward B. Roche ed., Amer. Pharma. Assoc., Pergamon Press (1987), and Protective Groups in Organic Chemistry, McOmie ed., Plenum Press, New York (1973). Examples of common esters include methyl, ethyl, trichloroethyl, propyl, butyl, pentyl, tert-butyl, benzyl, nitrobenzyl, methoxybenzyl and benzhydryl.

In some embodiments, a lipid comprises at least 10%, at least 20%, at least 30%, at least 35%, at least 40%, at least 50%, at least 60%, at least 70% or at least 80% by weight PUFA. In some embodiments, a lipid comprises at least 10%, at least 20%, at least 30%, at least 35%, at least 40%, at least 50%, at least 60%, at least 70% or at least 80% by weight DHA. In some embodiments, a lipid comprises less than 50%, less than 40%, less than 30%, less than 20%, less than 15%, less than 10%, or less than 5% by weight EPA. In some embodiments, a lipid comprises less than 10%, less than 5%, less than 2%, less than 1%, or less than 0.5% by weight sterols. In some embodiments, one or more PUFAs are present in a lipid in one or more forms, such as triglycerides, diglycerides, monoglycerides, phospholipids, free fatty acids, esterified fatty acids, alkali metal salts of fatty acids, alkali earth metal salts of fatty acids, and combinations thereof.

In some embodiments, a lipid separated after centrifuging in a process of the present invention comprises at least 50%, at least 60%, at least 70%, at least 80%, at least 90%, at least 95%, or 50% to 95%, 50% to 90%, 50% to 85%, 50% to 80%, 50% to 75%, 60% to 95%, 60% to 90%, 60% to 85%, 70% to 95%, 70% to 90%, 70% to 85%, 75% to 95%, 75% to 90%, or 75% to 85%, by weight of triglycerides.

In some embodiments, the triglycerides comprise at least 10%, at least 20%, at least 30%, at least 35%, at least 40%, at least 50%, at least 60%, at least 70% or at least 80% by weight DHA. In some embodiments, the triglycerides comprise at least 50%, at least 40%, at least 30%, at least 20%, at least 15%, at least 10%, or at least 5% by weight EPA.

As discussed herein, additional refining of a lipid after the centrifuging can provide a lipid comprising at least 80%, at least 85%, at least 90%, at least 95%, at least 99%, or 80% to 99.5%, 80% to 99%, 80% to 97%, 80% to 95%, 80% to 90%, 85% to 99.5%, 85% to 99%, 85% to 97%, 85% to 95%, 85% to 90%, 90% to 99.5%, 90% to 99%, 90% to 97%, 90% to 95%, 95% to 99.5%, 95% to 99%, 95% to 97%, 97% to 99.5%, or 98% to 99.5% triglyceride by weight.

As used herein, a "cell" refers to a lipid-containing biomaterial, such as biomaterial derived from microorganisms.

As used herein, a "microbial cell" or "microorganism" refers to organisms such as algae, bacteria, fungi, protist, and combinations thereof, e.g., unicellular organisms. In some embodiments, a microbial cell is a eukaryotic cell. A microbial cell suitable for use with the present invention includes, but is not limited to, golden algae (e.g., microorganisms of the kingdom Stramenopiles), green algae, diatoms, dinoflagellates (e.g., microorganisms of the order Dinophyceae including members of the genus Crypthecodinium such as, for example, *Crypthecodinium cohnii* or *C*. *cohnii*), yeast (Ascomycetes or Basidiomycetes), and fungi of the genera Mucor and Mortierella, including but not limited to *Mortierella alpina* and *Mortierella sect, schmuckeri.* A microbial cell suitable for use with the present invention can further include, but is not limited to genera found in the following groups of organisms: Stramenopiles, Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes, Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales.

In some embodiments, a microbial cell for use with the present invention is a microorganism of the phylum Labyrinthulomycota. In some embodiments, a microbial cell of the phylum Labyrinthulomycota is a thraustochytrid, such as a Schizochytrium or Thraustochytrium. According to the present invention, the term "thraustochytrid" refers to any member of the order Thraustochytriales, which includes the family Thraustochytriaceae, and the term "labyrinthulid" refers to any member of the order Labyrinthulales, which includes the family Labyrinthulaceae.

Members of the family Labyrinthulaceae were previously considered to be members of the order Thraustochytriales, but in more recent revisions of the taxonomic classification of such organisms, the family Labyrinthulaceae is now considered to be a member of the order Labyrinthulales. Both Labyrinthulales and Thraustochytriales are considered to be members of the phylum Labyrinthulomycota. Taxonomic theorists now generally place both of these groups of microorganisms with the algae or algae-like protists of the Stramenopile lineage.

For purposes of the present invention, strains of microbial cells described as thraustochytrids include the following organisms: Order: Thraustochytriales; Family: Thraustochytriaceae; Genera: Thraustochytrium (Species: sp., arudimentale, aureum, benthicola, globosum, kinnei, motivum, multirudimentale, pachydermum, proliferum, roseum, and striatum), Ulkenia (Species: sp., amoeboidea, kerguelensis, minuta, profunda, radiata, sailens, sarkariana, schizochytrops, visurgensis, yorkensis, and sp. BP-5601), Schizochytrium (Species: sp., aggregatum, limnaceum, mangrovei, minutum, and octosporum), Japonochytrium (Species: sp., marinam), Aplanochytrium (Species: sp., haliotidis, kerguelensis, profunda, and stocchinoi), Althornia (Species: sp., crouchii), or Elina (Species: sp., marisalba, and sinori*f*icd). For the purposes of this invention, species described within Ulkenia will be considered to be members of the genus Thraustochytrium. Aurantiacochytrium and Oblogospora are two additional genuses encompassed by the phylum Labyrinthulomycota in the present invention. In some embodiments, a microbial cell is of the genus Thraustochystrium, Schizochytrium, and mixtures thereof.

Microbial cells suitable for use with the present invention include, but are not limited to, Labyrinthulids selected from: Order: Labyrinthulales, Family: Labyrinthulaceae, Genera: Labyrinthula (Species: sp., algeriensis, coenocystis, chattonii, macrocystis, macrocystis atlantica, macrocystis, marina, minuta, *rosco f fensis,* valkanovii, vitellina, vitellina paci*f*ica, vitellina, and zop *f* ii), Labyrinthuloides (Species: sp., haliotidis, and yorkensis), Labyrinthomyxa (Species: sp., marina), Diplophrys (Species: sp., archeri), Pyrrhosorus (Species: sp., marinus), Sorodiplophrys (Species: sp., stercorea), and Chlamydomyxa (Species: sp., labyrinthuloides, and montana) (although there is currently not a consensus on the exact taxonomic placement of Pyrrhosorus, Sorodiplophrys, and Chlamydomyxa).

Host cells of the phylum Labyrinthulomycota include, but are not limited to, deposited strains PTA-10212, PTA-10213, PTA-10214, PTA-10215, PTA-9695, PTA-9696, PTA-9697, PTA-9698, PTA-10208, PTA-10209, PTA-10210, PTA-10211, the microorganism deposited as SAM2179 (named "Ulkenia SAME 179" by the depositor), any Thraustochytrium species (including former Ulkenia species such as *U visurgensis, U. amoeboida, U sarkariana, U profunda, U radiata, U. minuta* and Ulkenia sp. BP-5601), and including *Thraustochytrium striatum, Thraustochytrium aureum, Thraustochytrium roseum;* and any Japonochytrium species. Strains of Thraustochytriales include, but are not limited to Thraustochytrium sp. (23B) (ATCC *20891); Thraustochytrium striatum* (Schneider)(ATCC 24473); *Thraustochytrium aureum* (Goldstein) (ATCC 34304); *Thraustochytrium roseum* (Goldstein) (ATCC 28210); Japonochytrium sp. (LI) (ATCC 28207); ATCC 20890; ATCC 20892; a mutant strain derived from any of the aforementioned microorganisms; and mixtures thereof. Schizochytrium include, but are not limited to *Schizochytrium aggregatum, Schizochytrium limacinum,* Schizochytrium sp. (S31) (ATCC 20888), Schizochytrium sp. (S8) (ATCC 20889), Schizochytrium sp. (LC-RM) (ATCC 18915), Schizochytrium sp. (SR 21), deposited strain ATCC 28209, deposited *Schizochytrium limacinum* strain IFO 32693, a mutant strain derived from any of the aforementioned microorganisms, and mixtures thereof. In some embodiments, the host cell is a Schizochytrium or a Thraustochytrium. Schizochytrium can replicate both by successive bipartition and by forming sporangia, which ultimately release zoospores. Thraustochytrium, however, replicate only by forming sporangia, which then release zoospores. In some embodiments, the host cell of the invention is a recombinant host cell.

Effective culture conditions for a microbial cell for use with the invention include, but are not limited to, effective media, bioreactor, temperature, pH, and oxygen conditions that permit lipid production. An effective medium refers to any medium in which a microbial cell is typically cultured. Such media typically comprises an aqueous medium having assimilable carbon, nitrogen, and phosphate sources, as well as appropriate salts, minerals, metals, and other nutrients, such as vitamins. Microbial cells for use with the present invention can be cultured in conventional fermentation bioreactors, shake flasks, test tubes, microtiter dishes, and petri plates. In some embodiments, culturing is carried out at a temperature, pH, and oxygen content appropriate for a recombinant cell.

In some embodiments, a microbial cell is capable of growth at a salinity level of 12 g/L or less, 5 g/L or less, or 3 g/L or less of sodium chloride.

In some embodiments, a microbial cell produces a lipid comprising omega-3 and/or omega-6 PUFAs. In some embodiments, a microbial cell produces a lipid comprising DHA, DPA (n-3), DPA (n-6), EPA, arachidonic acid (ARA), or the like, and combinations thereof. Non-limiting examples of microorganisms that produce a lipid comprising a PUFA are disclosed above and are also found in U.S. Pat. Nos. 5,340,594, 5,340,742 and 5,583,019.

In some embodiments, a microbial cell comprises at least 30% by weight lipids, at least 35% by weight lipids, at least 40% by weight lipids, at least 50% by weight lipids, at least 60% by weight lipids, at least 70% by weight lipids, or at least 80% by weight lipids. In some embodiments, a microbial cell for use with the present invention is capable of producing at least 0.1 grams per liter per hour (g/L/h) of DHA, at least 0,2 g/L/h of DHA, at least 0.3 g/L/h of DHA, or at least 0.4 g/L/h of DHA.

### Processes

The processes of the present invention comprise concentrating a cell composition prior to lysis and demulsification. The concentration process comprises heating a composition comprising microbial cells, such as a fermentation broth, to a temperature of about 60°C to about 95 °C , about 80°C to about 95°C, about 85°C to about 90°C, about 90°C to about 95°C, about 80°C, about 85°C, about 90°C, about 95°C, or higher, at a pH of about 6 to about 9, about 9 to about 11, about 9 to about 10.5, about 9 to about 10, or about 9 to about 9.5 under agitation for a period of time from about 1 hour, 2 hours, 3 hours, 4 hours, 5 hours, 6 hours or more to concentrate the population of microbial cells; and separating the concentrated microbial cells into a light phase and a heavy phase.

The processes of the present invention further comprise lysing a cell or cell biomass from the light phase to form a lysed cell composition. As used herein, the term "cell biomass" refers to a population of microbial cells. As used herein, the terms "lyse" and "lysing" refer to a process of rupturing the cell wall and/or cell membrane of a cell. In some embodiments, lysing comprises a process such as: mechanically treating, chemically treating, enzymatically treating, physically treating, or combinations thereof. In various embodiments, the lysis occurs by the addition of an enzyme capable of disrupting the cell wall of the microbial cells.

As used herein, "mechanically treating" includes, but is not limited to, homogenizing a cell, applying ultrasound to a cell, cold-pressing a cell, milling a cell or the like, and combinations thereof. In some embodiments, the process comprises lysing the cell by homogenization. In some embodiments, the process comprises lysing the cell with a homogenizer.

In some embodiments, the concentration step comprises a mechanical process such as homogenization or sonication wherein the mechanical process results in lysis of less than about 5% of the cells.

Homogenizing a cell can include, but is not limited to, processes utilizing a French pressure cell press, a sonicator, a homogenizer, a ball mill, a rod mill, a pebble mill, a bead mill, a high-pressure grinding roll, a vertical shaft impactor, an industrial blender, a high shear mixer, a paddle mixer, a polytron homogenizer or the like, and combinations thereof. In some embodiments, a cell is flowed through a homogenizer that is optionally heated. In some embodiments, suitable homogenization can include 1 to 3 passes through a homogenizer at either high and/or low pressures. In some embodiments, a pressure during homogenization can be 150 bar to 1,400 bar, 150 bar to 1,200 bar, 150 bar to 900 bar, 150 bar to 300 bar, 300 bar to 1,400 bar, 300 bar to 1,200 bar, 300 bar to 900 bar, 400 bar to 800 bar, 500 bar to 700 bar, or 600 bar.

As used herein, physically treating can include, but is not limited to, heating a cell, cooling a cell, drying a cell, or the like, and combinations thereof.

Heating a cell can include, but is not limited to, resistive heating, convection heating, steam heating, heating in a fluid bath, heating with solar energy, heating with focused solar energy, and the like, any of which can be performed in a tank, pool, tube, conduit, flask, or other containment device. In some embodiments, a cell is heated in a tank that includes resistive coils in/on its walls. In some embodiments, a cell is heated in a liquid bath that includes a tubing passing there through. In some embodiments, a cell is heated in a tank with jacketed heating, which is tank that uses a heating "jacket" around the tank through which a heating fluid is circulated.

Drying a cell can include, but is not limited to, exposing to air flow, exposing to heat (e.g., convection heat, a heated surface, and the like), exposing to solar energy, freeze drying (lyophilizing), spray drying, and combinations thereof. In some embodiments, drying comprises applying a cell to a rotating drum that is optionally heated.

As used herein, chemically treating includes, but is not limited to, raising or lowering the pH of a cell or cell composition, contacting a cell or cell composition with a suitable chemical or the like.

Raising the pH of a cell or cell composition can include, but is not limited to, adding a base to a cell composition. In some embodiments, bases suitable for use with the present invention include, but are not limited to, hydroxide bases (e.g., LiOH, NaOH, KOH, Ca(OH)₂, and the like, and combinations thereof), carbonate bases (e.g., Na₂CO₃, K₂CO₃, MgCO₃, and the like, and combinations thereof), bicarbonate bases (e.g., LiHCO₃, NaHCO₃, KHCO₃, and the like, and combinations thereof), and combinations thereof. A base can be in the form of a solid (e.g., crystals, a granulate, pellets, and the like) or a liquid (e.g., an aqueous solution, an alcoholic solution such as a hydroxide base in methanol, ethanol, propanol, and the like), and combinations thereof. In some embodiments, the pH of the cell composition is raised to 8 or above, 9 or above, 10 or above, 11 or above, 12 or above, or a pH of 7 to 13, 7 to 12, 7 to 11 , 7 to 10, 7 to 9, 8 to 13, 8 to 12, 8 to 11 , 8 to 10, 8 to 9, 9 to 12, 9 to 11, 9 to 10, 10 to 12, or 10 to 11.

In some embodiments, raising a pH of a cell can include, but is not limited to, performing a chloralkali process. In some embodiments, a fermentation broth containing sodium chloride and a cell composition is subjected to electrolysis, which would result in the formation of sodium hydroxide. The formation of sodium hydroxide raises the pH of the cell. In some embodiments, a fermentation broth can include calcium chloride or potassium chloride in place of or in addition to sodium chloride. Subjecting such a fermentation broth to electrolysis results in the formation of calcium hydroxide or potassium hydroxide, respectively, thereby raising the pH of the cell.

Enzymatic lysing refers to lysis of a cell wall or cell membrane of a cell by contacting the cell with one or more enzymes. Enzymes suitable for use with the present invention include, but are not limited to, beta-glucanases, xylanases, proteases, mannanases, cellulases, hemicellulases, chitinases, pectinases, and combinations thereof. Non-limiting examples of proteases include serine proteases, threonine proteases, cysteine proteases, aspartate proteases, metalloproteases, glutamic acid proteases, alacase, and combinations thereof. Non-limiting examples of cellulases include sucrase, maltase, lactase, alpha-glucosidase, beta-glucosidase, amylase, lysozyme, neuraminidase, galactosidase, alpha- mannosidase, glucuronidase, hyaluronidase, pullulanase, glucocerebrosidase, galactosylceramidase, acetylgalactosaminidase, fucosidase, hexosaminidase, iduronidase, maltase-glucoamylase, and combinations thereof. A non-limiting example of a chitinase includes chitotriosidase. Non-limiting examples of pectinases include pectolyase, pectozyme, polygalacturonase, and combinations thereof. In some embodiments, some enzymes are activated by heating. In some embodiments, the enzyme is selected from beta-glucanase,
xylanase, cellulase, protease, pectinase, mannanase, amylase, and combinations thereof. In some embodiments, the enzyme is Alcalase^{®}.

As used herein, a "lysed cell composition" refers to a composition comprising one or more lysed cells, including cell debris and other contents of the cell, in combination with a lipid (from the lysed cells), and optionally, broth that contains microbial cells. In some embodiments, a microbial cell is contained in a fermentation broth or media comprising the microbial cell and water. In some embodiments, a lysed cell composition refers to a composition comprising one or more lysed cells, cell debris, a lipid, the natural contents of the cell, and aqueous components from a broth. In some embodiments, a lysed cell composition is in the form of an oil-in-water emulsion comprising a mixture of a continuous aqueous phase and a dispersed lipid phase. In some embodiments, a dispersed lipid phase is present in a concentration of 1% to 60%, 1% to 50%, 1% to 40%, 1% to 30%, 1% to 20%, 5% to 60%, 5% to 50%, 5% to 40%, 5% to 30%, 5% to 20%, 10% to 60%, 10% to 50%, 10% to 40%, 20% to 60%, 20% to 50%, 20% to 40%, 30% to 60%, 30% to 50%, or 40% to 60% by weight of an emulsified lysed cell composition.

While not being bound to any particular theory, it is believed the processes of the present invention break up or demulsify an emulsified lysed cell composition, allowing a lipid to be separated from the lysed cell composition. As used herein, the terms "emulsion" and "emulsified" refers to a mixture of two or more immiscible phases or layers wherein one phase or layer is dispersed in another phase or layer. As used herein, the terms "break," "break up," "demulsify," "demulsification," "demulsifying," and "breaking" refer to a process of separating immiscible phases or layers of an emulsion. For example, demulsifying or breaking an emulsified lysed cell composition refers to a process by which an emulsified lysed cell composition changes from an emulsion having one or more phases or layers to a composition having two or more phases or layers. For example, in some embodiments, the process of the present invention breaks an emulsified lysed cell composition from a single-phase to two or more phases. In some embodiments, the two or more phases include a lipid phase and an aqueous phase. In some embodiments, the process of the present invention breaks an emulsified lysed cell composition from one or more phases to at least three phases. In some embodiments, the three phases include a lipid phase, an aqueous phase, and a solid phase. In some embodiments, the three phases include a lipid phase, an emulsion phase, and an aqueous phase.

In some embodiments, the processes of the present invention demulsify a lysed cell composition to form a demulsified cell composition by removing or breaking at least 75% of the emulsion, at least 80% of the emulsion, at least 85% of the emulsion, at least 90% of the emulsion, at least 95% of the emulsion, at least 99% of the emulsion. In some embodiments, the process of the present invention demulsify a lysed cell composition by removing or breaking 75% of the emulsion to 99% of the emulsion, 75% of the emulsion to 95% of the emulsion, 75% of the emulsion to 90% of the emulsion, 75% of the emulsion to 85% of the emulsion, 75% of the emulsion to 80% of the emulsion, 80% of the emulsion to 99% of the emulsion, 80% of the emulsion to 95% of the emulsion, 80% of the emulsion to 90% of the emulsion, 80% of the emulsion to 85% of the emulsion, 85% of the emulsion to 99% of the emulsion, 85% of the emulsion to 95% of the emulsion, 85% of the emulsion to 90% of the emulsion, 90% of the emulsion to 99% of the emulsion, 90% of the emulsion to 95% of the emulsion, or 95% of the emulsion to 99% of the emulsion by weight or volume.

In some embodiments, prior to concentrating the cell composition, the cell composition can be washed and/or pasteurized.

In some embodiments, washing the cell includes using an aqueous solution, such as water, to remove any extracellular water-soluble or water-dispersible compounds. In some embodiments, the cell can be washed once, twice, thrice, or more. In some embodiments, pasteurizing the cell includes heating the cell to a temperature sufficient to inactivate any undesirable enzymes or to activate any desirable enzymes, for example any enzymes that might degrade lipid or reduce the yield of PUFAs. In some embodiments, the pasteurization temperature is about 60°C to about 80°C. In some embodiments, the cell can be washed first and then pasteurized.

In some embodiments, treating a lysed cell composition with at least a first base and at least a first acid breaks up (i.e., demulsifies) an emulsified lysed cell composition. In some embodiments, treating a lysed cell composition with a salt breaks (i.e., demulsifies) an emulsified lysed cell composition. In some embodiments, heating a lysed cell composition breaks (i.e., demulsifies) an emulsified lysed cell composition. In some embodiments, agitating a lysed cell composition breaks (i.e., demulsifies) an emulsified lysed cell composition. In some embodiments, simultaneous heating and agitating of a lysed cell composition breaks (i.e., demulsifies) an emulsified lysed cell composition. In some embodiments, one or more of the preceding treatments breaks up (i.e., demulsifies) an emulsified lysed cell composition.

In some embodiments, the process of the invention comprises raising the pH of a cell composition to lyse and/or demulsify the cell composition. In some embodiments, the process of the invention comprises raising the pH of a lysed cell composition to demulsify the lysed cell composition. In some embodiments, raising the pH comprises contacting a cell composition or lysed cell composition with a base. In some embodiments, the process of the invention comprises contacting a lysed cell composition with a base to demulsify the lysed cell composition. As used herein, "contacting" refers to combining a cell composition or a lysed cell composition with a second composition (e.g., by adding a composition to a cell composition or a lysed cell composition, by adding a cell composition or a lysed cell composition to a composition, and the like). As used herein, a "composition" can comprise a pure material or include a combination of two or more materials, substances, excipients, portions, and the like. Contacting a lysed cell composition with a first base raises the pH of the lysed cell composition.

In some embodiments, a lysed cell composition is contacted with a first base for a period of time followed by a first acid for a period of time, then heated, agitated, or a combination thereof, and subsequently contacted with a second or subsequent base followed by a second or subsequent acid to provide a demulsified composition.

In some embodiments, the base has a pK_{b} of 1 to 12, 1 to 10, 1 to 8, 1 to 6, 1 to 5, 2 to 12,2 to 10,2 to 8,2 to 6, 2 to 5, 3 to 10, 3 to 6, 3 to 5, 4 to 10,4 to 8, 4 to 6, 5 to 10, or 5 to 8. As used herein, the term "pK_{b}" refers to the negative logarithm of the base association constant, K_{b}, of the base. K_{b} refers to the equilibrium constant for the ionization of the base in water.

Bases suitable for use with the present invention include, but are not limited to, hydroxide bases (e.g., LiOH, NaOH, KOH, Ca(OH)₂, and the like, and combinations thereof), carbonate bases (e.g., Na₂CO₃, K₂CO₃, MgCO₃, and the like, and combinations thereof), bicarbonate bases (e.g., LiHCO₃, NaHCO₃, KHCO₃, and the like, and combinations thereof), and combinations thereof.

A base can be in the form of a solid (e.g., crystals, a granulate, pellets, and the like) or a liquid (e.g., an aqueous solution, an alcoholic solution such as a hydroxide base in methanol, ethanol, propanol, and the like), and combinations thereof. Thus, a solvent can be optionally present in a base for use with the present invention. As used herein, "solvent" refers to an agent that is hydrophobic or lipophilic. As used herein, "hydrophobic" refers to an agent that is repelled from a mass of water. As used herein, "lipophilic" refers to an agent that dissolves in lipids.

In some embodiments, contacting a cell composition or a lysed cell composition with a base raises the pH of the lysed cell composition. In some embodiments, contacting a lysed cell composition with a base raises the pH of the lysed cell composition to 9 or above, 10 or above, 11 or above, 12 or above, or a pH of from about 9 to about 14, about 9 to about 13.5, about 9 to about 13, about 9 to about 12.5, about 9 to about 12, about 9 to about 11.5, about 9 to about 11, about 9 to about 10.5, about 9 to about 10, about 9 to about 9.5, about 9.5 to about 14, about 9.5 to about 13.5, about 9.5 to about 13, about 9.5 to about 12.5, about 9.5 to about 12, about 9.5 to about 11.5, about 9.5 to about 11, about 9.5 to about 10.5, about 9.5 to about 10, about 10 to about 14, about 10 to about 13.5, about 10 to about 13, about 10 to about 12.5, about 10 to about 12, about 10 to about 11.5, about 10 to about 11, about 10 to about 10.5, about 10.5 to about 14, about 10.5 to about 13.5, about 10.5 to about 13, about 10.5 to about 12.5, about 10.5 to about 12, about 10.5 to about 11.5, about 10.5 to about 11, about 11 to about 14, about 11 to about 13.5, about 11 to about 13, about 11 to about 12.5, about 11 to about 12, about 11 to about 11.5, about 11.5 to about 14, about 11.5 to about 13.5, about 11.5 to about 13, about 11.5 to about 12.5, about 11.5 to about 12, about 12 to about 14, about 12 to about 13.5, about 12 to about 13, about 12 to about 12.5, about 12.5 to about 14, about 12.5 to about 13.5, about 12.5 to about 13, about 13 to about 14, about 13 to about 13.5, or about 13.5 to about 14.

In some embodiments, a base is added in an amount of about 2% to about 10%, about 2% to about 9%, about 2% to about 8%, about 2% to about 7%, about 2% to about 6%, about 3% to about 6%, about 4% to about 6%, about 5% to about 6%, about 2% to about 5%, about 2% to about 4%, about 2% to about 3%, about 3% to about 5%, about 3% to about 4%, or about 4% to about 5% by weight (or volume) of the cell broth to raise the pH.

In some embodiments, the pH is lowered by adding an acid. In some embodiments, the acid has a pKₐ of 1 to 12, 1 to 10, 1 to 8, 1 to 6, 1 to 5, 2 to 12, 2 to 10, 2 to 8, 2 to 6, 2 to 5, 3 to 10, 3 to 6, 3 to 5, 4 to 10, 4 to 8, 4 to 6, 5 to 10, or 5 to 8. As used herein, the term "pKₐ" refers to the negative logarithm of the base association constant, Kₐ, of the acid. Kₐ refers to the equilibrium constant for the ionization of the acid in water.

The acids include, but are not limited to, sulfuric; phosphoric; hydrochloric; hydrobromic; hydroiodic; hypochlorous; chlorous; chloric; perchloric; fluorosulfuric; nitric; fluoroantimonic; fluoroboric; hexafluorophosphoric; chromic; boric; acetic; citric; formic; and combinations thereof. In some embodiments, the pH is selected from about 6.5 or below; about 6 or below; about 5.5 or below; about 5 or below; about 4.5 or below; about 4 or below; about 3.5 or below; about 3 or below; about 2.5 or below; about 2 or below; about 1.5 or below; about 1 or below; and about 0.5 or below. In other embodiments, the pH is selected from about 0.5 to about 6.5; about 0.5 to about 6; about 0.5 to about 5.5; about 0.5 to about 5; about 0.5 to about 4.5; about 0.5 to about 4; about 0.5 to about 3.5; about 0.5 to about 3; about 0.5 to about 2.5; about 0.5 to about 2; about 0.5 to about 1.5; about 0.5 to about 1; about 1 to about 7; about 1 to about 6.5; about 1 to about 6; about 1 to about 5.5; about 1 to about 5; about 1 to about 4.5; about 1 to about 4; about 1 to about 3.5; about 1 to about 3; about 1 to about 2.5; about 1 to about 2; about 1 to about 1.5; about 1.5 to about 7; about 1.5 to about 6.5; about 1.5 to about 6; about 1.5 to about 5.5; about 1.5 to about 5; about 1.5 to about 4.5; about 1.5 to about 4; about 1.5 to about 3.5; about 1.5 to about 3; about 1.5 to about 2.5; about 1.5 to about 2; about 2 to about 7; about 2 to about 6.5; about 2 to about 6; about 2 to about 5.5; about 2 to about 5; about 2 to about 4.5; about 2 to about 4, about 2 to about 3.5; about 2 to about 3; about 2 to about 2.5; about 2.5 to about 7; about 2.5 to about 6.5; about 2.5 to about 6; about 2.5 to about 5.5; about 2.5 to about 5; about 2.5 to about 4.5; about 2.5 to about 4; about 2.5 to about 3.5; about 2.5 to about 3; about 3 to about 7; about 3 to about 6.5; about 3 to about 6; about 3 to about 5.5; about 3 to about 5; about 3 to about 4.5; about 3 to about 4; about 3 to about 3.5; about 3.5 to about 7; about 3.5 to about 6.5; about 3.5 to about 6; about 3.5 to about 5.5; about 3.5 to about 5; about 3.5 to about 4.5; about 3.5 to about 4; about 4 to about 7; about 4 to about 6.5; about 4 to about 6; about 4 to about 5.5; about 4 to about 5; about 4 to about 4.5; about 4.5 to about 7; about 4.5 to about 6.5; about 4.5 to about 6; about 4.5 to about 5.5; about 4.5 to about 5; about 5 to about 7; about 5 to about 6.5; about 5 to about 6; about 5 to about 5.5; about 5.5 to about 7; about 5.5 to about 6.5; about 5.5 to about 6; and about 6 to about 6.5.

In some embodiments, an acid is added in an amount of about 0.5% to about 1%, about 1% to about 2%, about 2% to about 10%, about 2% to about 9%, about 2% to about 8%, about 2% to about 7%, about 2% to about 6%, about 3% to about 6%, about 4% to about 6%, about 5% to about 6%, about 2% to about 5%, about 2% to about 4%, about 2% to about 3%, about 3% to about 5%, about 3% to about 4%, or about 4% to about 5% by weight (or volume) of the cell broth to lower the pH.

In some embodiments, the process comprises contacting a concentrated cell composition or lysed cell composition with a salt to facilitate the demulsification of the lysed cell composition. As used herein, a "salt" refers to an ionic compound formed by replacing a hydrogen ion from an acid with a metal (e.g., an alkali metal, an alkali earth metal, a transition metal, and the like) or a positively charged compound (e.g., NH₄⁺ and the like). Salts suitable for use with the present invention include, but are not limited to, alkali metal salts, alkali earth metal salts, or the like, and combinations thereof. Negatively charged ionic species present in a salt for use with the present include, but are not limited to, halides, sulfate, bisulfate, sulfite, phosphate, hydrogen phosphate, dihydrogen phosphate, carbonate, bicarbonate, or the like, and combinations thereof. In some embodiments, a salt for use with the present invention is selected from: sodium chloride, sodium sulfate, sodium carbonate, calcium chloride, potassium sulfate, magnesium sulfate, monosodium glutamate, ammonium sulfate, potassium chloride, iron chloride, iron sulfate, aluminum sulfate, and combinations thereof. In some embodiments, a salt does not include NaOH. A salt can be added as a solid (e.g., in crystalline, amorphous, pelletized, and/or granulated form), and/or as a solution (e.g., a dilute solution, a saturated solution, or a super-saturated solution) containing, for example, water, an alcohol, and the like, and combinations thereof.

In some embodiments, the salt is added in an amount of 5 g/l to 25 g/l, 5 g/l to 10 g/l, 10 g/l to 15 g/l, 15 g/l to 20 g/l, 20 g/l to 25 g/l, or 10 g/l to 20 g/l.

In some embodiments, the temperature of the concentrated cell composition or the lysed cell composition is less than or equal to 60°C, less than or equal to 55°C, less than or equal to 45°C, less than or equal to 40°C, less than or equal to 35°C, less than or equal to 30°C, or less than or equal to 25°C when a salt is added to demulsify the cell composition or the lysed cell composition. In some embodiments, the temperature of the lysed cell composition is 0°C to 60°C, 0°C to 55°C, 0°C to 50°C, 0°C to 45°C, 0°C to 40°C, 0°C to 35°C, 0°C to 30°C, 0°C to 25°C, 20°C to 60°C, 20°C to 55°C, 20°C to 50°C, 20°C to 45°C, 20°C to 40°C, 20°C to 35°C, 20°C to 30°C, 30°C to 60°C, 30°C to 55°C, 30°C to 50°C, 30°C to 45°C, 30°C to 40°C, 30°C to 40°C, 40°C to 60°C, 40°C to 55°C, 40°C to 50°C, or 50°C to 60°C when a salt is added to demulsify the cell composition or the lysed cell composition.

In some embodiments, the process comprises a temperature shock cycle before, during, or after one or more of the pH shock cycles. A "temperature shock cycle" refers herein to a cycle where the cell composition or lysed cell composition is raised to a temperature of about 80°C, about 85°C, about 90°C, about 95°C, or higher, for period of time (e.g., about 5 minutes, about 10 minutes, about 20 minutes, about 30 minutes, about 50 minutes, about one hour, about 1.5 hours, about 2 hours, about 2.5 hours, about 3 hours or longer) then lowered to a temperature of about 30°C, about 25°C, about 20°C, about 15°C, about 10°C, about 5°C, or about 4°C for a period of time (e.g., about 5 minutes, about 10 minutes, about 20 minutes, about 30 minutes, about 50 minutes, about one hour, about 1.5 hours, about 2 hours, about 2.5 hours, about 3 hours or longer) to facilitate demulsification. In some embodiments, the temperature shock cycle occurs before the pH shock cycle. In other embodiments, the temperature shock cycle occurs after the pH shock cycle. In yet other embodiments, the temperature shock cycle occurs between two or more of the pH shock cycles.

In some embodiments, the process comprises contacting a concentrated cell composition or a lysed cell composition with 20% or less, 15% or less, 10% or less, 7.5% or less, 5% or less, or 2% or less salt by weight, of the lysed cell composition or the cell composition. In some embodiments, a process comprises contacting a concentrated cell composition or a lysed cell composition with 0.1% to 20%, 0.1% to 15%, 0.1% to 10%, 0.5% to 20%, 0.5% to 15%, 0.5% to 10%, 0.5% to 5%, 0.5% to 4%, 0.5% to 3%, 0.5% to 2.5%, 0.5% to 2%, 0.5% to 1.5%, 0.5% to 1%, 1% to 20%, 1% to 15%, 1% to 10%,1% to 5%, 1% to 4%, 1% to 3%, 1% to 2.5%, 1% to 2%, 1% to 1.5%, 1.5% to 5%, 1.5% to 4%, 1.5% to 3%, 1.5% to 2.5%, 1.5% to 2%, 2% to 20%, 2% to 15%, 2% to 10%, 2% to 5%, 2% to 4%, 2% to 3%, 2% to 2.5%, 2.5% to 5%, 2.5% to 4%, 2.5% to 3%, 3% to 5%, 3% to 4%, 4% to 5%, 5% to 20%, 5% to 15%, 5% to 10%, 10% to 20%, 10% to 15%, or 15% to 20% salt, by weight, of the concentrated cell composition or lysed cell composition (e.g., a total broth weight). For example, when a lysed cell composition weighs 1,000 kg, contacting with 0.5% to 20% salt, by weight, requires combining 5 kg to 200 kg of salt with the lysed cell composition.

In some embodiments, the process comprises heating a concentrated cell composition or a lysed cell composition to demulsify the lysed cell composition. In some embodiments the cell composition or the lysed cell composition is heated for a sufficient period of time for a base and/or a salt to demulsify a cell composition or a lysed cell composition. In some embodiments, the process comprises heating a cell composition or a lysed cell composition for at least 5 minutes, at least 10 minutes, at least 20 minutes, at least 30 minutes, at least 1 hour, at least 2 hours, at least 4 hours, at least 8 hours, at least 12 hours, at least 18 hours, at least 24 hours, at least 30 hours, at least 36 hours, at least 42 hours, at least 48 hours, at least 54 hours, at least 60 hours, at least 66 hours, at least 72 hours, at least 78 hours, at least 84 hours, at least 90 hours or at least 96 hours. In some embodiments, the process comprises heating a lysed cell composition for 5 minutes to 96 hours, 5 minutes to 4 hours, 5 minutes to 2 hours, 5 minutes to 1 hour, 10 minutes to 4 hours, 10 minutes to 2 hours, 10 minutes to 1 hour, 1 hour to 2 hours, 1 hour to 96 hours, 1 hour to 84 hours, 1 hour to 72 hours, 1 hour to 60 hours, 1 hour to 48 hours, 1 hour to 36 hours, 1 hour to 24 hours, 1 hour to 4 hours, 4 hours to 96 hours, 4 hours to 84 hours, 4 hours to 72 hours, 4 hours to 60 hours, 4 hours to 48 hours, 4 hours to 36 hours, 4 hours to 24 hours, 8 hours to 96 hours, 8 hours to 84 hours, 8 hours to 72 hours, 8 hours to 60 hours, 8 hours to 48 hours, 8 hours to 36 hours, 8 hours to 24 hours, 8 hours to 12 hours, 12 hours to 96 hours, 12 hours to 84 hours, 12 hours to 72 hours, 12 hours to 60 hours, 12 hours to 48 hours, 12 hours to 36 hours, 12 hours to 24 hours, 24 hours to 96 hours, 24 hours to 84 hours, 24 hours to 72 hours, 24 hours to 60 hours, 24 hours to 48 hours, or 24 hours to 36 hours.

In some embodiments, a cell composition or a lysed cell composition can be heated at a temperature of at least 10°C, at least 20°C, at least 25°C, at least 30°C, at least 35°C, at least 40°C, at least 45°C, at least 50°C, at least 55°C, at least 60°C, at least 65°C, at least 70°C, at least 75°C, at least 80°C, at least 85°C, at least 90°C, at least 95°C, or at least 100°C. In some embodiments, a process comprises heating a cell composition or a lysed cell composition at a temperature of 10°C to 100°C, 10°C to 90°C, 10°C to 80°C, 10°C to 70°C, 20°C to 100°C, 20°C to 90°C, 20°C to 80°C, 20°C to 70°C, 30°C to 100°C, 30°C to 90°C, 30°C to 80°C, 30°C to 70°C, 40°C to 100°C, 40°C to 90°C, 40°C to 80°C, 50°C to 100°C, 50°C to 90°C, 50°C to 80°C, 50°C to 70°C, 60°C to 100°C, 60°C to 90°C, 60°C to 80°C, 70°C to 100°C, 70°C to 90°C, 80°C to 100°C, 80°C to 90°C, or 90°C to 100°C. In some embodiments, a salt can be added to the cell composition or the lysed cell composition during the heating. In various embodiments, coagulating agents such as aluminum sulfate and chitosan can be added to the cell composition to facilitate the concentration step. In these embodiments, the concentrated biomass separates into the heavy phase, whereas the light phase consists primarily of aqueous material.

In some embodiments, a cell composition or a lysed cell composition can be heated in a closed system or in a system with an evaporator. In some embodiments, a cell composition or lysed cell composition can be heated in a system with an evaporator such that a portion of the water present in the cell composition or the lysed cell composition is removed by evaporation. In some embodiments, a process comprises heating a cell composition or a lysed cell composition in a system with an evaporator to remove up to 1%, 5%, 10%, 15%, 20%, 25%, 30%, 35%, 40%, 45% or 50% by weight of water present in the cell composition or lysed cell composition. In some embodiments, a process comprises heating a cell composition or a lysed cell composition in a system with an evaporator to remove 1% to 50%, 1% to 45%, 1% to 40%, 1% to 35%, 1% to 30%, 1% to 25%, 1% to 20%, 1% to 15%, 1% to 10%, 1% to 5%, 5% to 50%, 5% to 45%, 5% to 40%, 5% to 35%, 5% to 30%, 5% to 25%, 5% to 20%, 5% to 15%, 5% to 10%, 10% to 50%, 10% to 45%, 10% to 40%, 10% to 35%, 10% to 30%, 10% to 25%, 10% to 20%, 10% to 15%, 15% to 50%, 15% to 45%, 15% to 40%, 15% to 35%, 15% to 30%, 15% to 25%, 15% to 20%, 20% to 50%, 20% to 45%, 20% to 40%, 20% to 35%, 20% to 30%, 20% to 25%, 25% to 50%, 25% to 45%, 25% to 40%, 25% to 35%, 25% to 30%, 30% to 50%, 30% to 45%, 30% to 40%, 30% to 35%, 35% to 50%, 35% to 45%, 35% to 40%, 40% to 50%, 40% to 45%, or 45% to 50%.

In some embodiments, the process comprises holding a concentrated cell composition or a lysed cell composition in a vessel for a predetermined time to demulsify the lysed cell composition. In some embodiments, the process comprises holding a cell composition or a lysed cell composition in a vessel for at least 5 minutes, at least 10 minutes, at least 20 minutes, at least 30 minutes, at least 1 hour, at least 2 hours, at least 4 hours, at least 8 hours, at least 12 hours, at least 18 hours, at least 24 hours, at least 30 hours, at least 36 hours, at least 42 hours, at least 48 hours, at least 54 hours, at least 60 hours, at least 66 hours, at least 72 hours, at least 78 hours, at least 84 hours, at least 90 hours or at least 96 hours. In some embodiments, the process comprises holding a cell composition or a lysed cell composition at each of the pH levels in the cycle for 5 minutes to 96 hours, 5 minutes to 4 hours, 5 minutes to 2 hours, 5 minutes to 1 hour, 10 minutes to 4 hours, 10 minutes to 2 hours, 10 minutes to 1 hour, 1 hour to 96 hours, 1 hour to 84 hours, 1 hour to 72 hours, 1 hour to 60 hours, 1 hour to 48 hours, 1 hour to 36 hours, 1 hour to 24 hours, 1 hour to 4 hours, 1 hour to 2 hours, 4 hours to 96 hours, 4 hours to 84 hours, 4 hours to 72 hours, 4 hours to 60 hours, 4 hours to 48 hours, 4 hours to 36 hours, 4 hours to 24 hours, 8 hours to 96 hours, 8 hours to 84 hours, 8 hours to 72 hours, 8 hours to 60 hours, 8 hours to 48 hours, 8 hours to 36 hours, 8 hours to 24 hours, 8 hours to 12 hours, 12 hours to 96 hours, 12 hours to 84 hours, 12 hours to 72 hours, 12 hours to 60 hours, 12 hours to 48 hours, 12 hours to 36 hours, 12 hours to 24 hours, 24 hours to 96 hours, 24 hours to 84 hours, 24 hours to 72 hours, 24 hours to 60 hours, 24 hours to 48 hours, or 24 hours to 36 hours.

In some embodiments, the process comprises contacting a lysed cell emulsion with an antioxidant, optionally before and/or after pasteurization. Antioxidants suitable for use with the present invention include, but are not limited to, a tocopherol, a tocotrienol, a polyphenol, resveratrol, a flavonoid, a carotenoid, lycopene, a carotene, lutein, ascorbic acid, ascorbyl palmitate, or the like, and combinations thereof.
As used herein, the terms "agitating" and "agitation" refer to a process of affecting motion in a lysed cell composition through an application of force. In some embodiments, the process of the invention comprises agitating a cell composition or a lysed cell composition by stirring, mixing, blending, shaking, vibrating, or a combination thereof. In some embodiments, the process of agitating a cell composition or a lysed cell composition demulsifies the cell composition or the lysed cell composition.

In some embodiments, the process of the invention comprises agitating a cell composition or a lysed cell composition at 74.56 watt /3785 litre (0.1 hp/1000 gal) to 7456 watt /3785 litre (10 hp/1000 gal), 370 watt /3785 litre (0.5 hp/1000 gal) to 5965.6 watt /3785 litre (8 hp/1000 gal), 745.6 watt /3785 litre (1 hp/1000 gal) to 4474.2 watt /3785 litre (6 hp/1000 gal) or 1491.4 watt /3785 litre (2 hp/1000 agl) to 3728.5 watt /3785 litre (5 hp/1000 gal) of lysed cell composition.

In some embodiments, the process of the invention comprises agitating a cell composition or a lysed cell composition using an agitator. In some embodiments, the agitator is a dispersion style agitator that disperses a base and/or salt in the cell composition or the lysed cell composition. In some embodiments, an agitator has one or more impellers. As used herein, "impeller" refers to a device arranged to impart motion to a cell composition or a lysed cell composition when rotated. Impellers suitable for use with the present invention include straight blade impellers, Rushton blade impellers, axial flow impellers, radial flow impellers, concave blade disc impellers, high-efficiency impellers, propellers, paddles, turbines, or the like, and combinations thereof. In some embodiments, a process includes agitating a cell composition or a lysed cell composition using an agitator having an impeller tip speed of 0.4572 m/s (90 ft/min) to 6.096 m/s (1200 ft/min), 1.016 m/s (200 ft/min) to 5.08 m/s (1000 ft/min), 1.524 m/s (300 ft/min) to 4.064 m/s (800 ft/min), 2.032 m/s (400 ft/min) to 3.556 m/s (700 ft/min) or 2.54 m/s (500 ft/min) to 3.048 m/s (600 ft/min). In some embodiments, a process includes agitating a cell composition or a lysed cell composition using an agitator having an impeller tip speed of 350 centimeters/second to 900 centimeters per second, 350 centimeters/second to 850 centimeters per second, 350 centimeters/second to 800 centimeters/second, 350 centimeters/second to 750 centimeters/second, 350 centimeter s/second to 700 centimeters/second, 350 centimeters/second to 650 centimeters/second, 350 centimeters/second to 600 centimeters/second, 350 centimeters/second to 550 centimeters/second, 350 centimeters/second to 500 centimeters/second, 350 centimeters/second to 450 centimeters/second, 350 centimeter s/second to 400 centimeter s/second, 400 centimeters/second to 900 centimeters per second, 400 centimeters/second to 850 centimeters per second, 400 centimeters/second to 800 centimeters/second, 400 centimeters/second to 750 centimeters/second, 400 centimeters/second to 700 centimeters/second, 400 centimeter s/second to 650 centimeters/second, 400 centimeters/second to 600 centimeters/second, 400 centimeters/second to 550 centimeters/second, 400 centimeters/second to 500 centimeters/second, 400 centimeters/second to 450 centimeters/second, 450 centimeters/second to 900 centimeters per second, 450 centimeters/second to 850 centimeters per second, 450 centimeters/second to 800 centimeters/second, 450 centimeters/second to 750 centimeters/second, 450 centimeters/second to 700 centimeters/second, 450 centimeters/second to 650 centimeters/second, 450 centimeters/second to 600 centimeters/second, 450 centimeters/second to 550 centimeters/second, 450 centimeters/second to 500 centimeters/second, 500 centimeters/second to 900 centimeters per second, 500 centimeters/second to 850 centimeters per second, 500 centimeters/second to 800 centimeters/second, 500 centimeters/second to 750 centimeters/second, 500 centimeters/second to 700 centimeters/second, 500 centimeter s/second to 650 centimeters/second, 500 centimeters/second to 600 centimeters/second, 500 centimeters/second to 550 centimeters/second, 550 centimeters/second to 900 centimeters per second, 550 centimeters/second to 850 centimeters per second, 550 centimeters/second to 800 centimeters/second, 550 centimeters/second to 750 centimeters/second, 550 centimeters/second to 700 centimeters/second, 550 centimeters/second to 650 centimeters/second, 550 centimeters/second to 600 centimeters/second, 600 centimeters/second to 900 centimeters per second, 600 centimeters/second to 850 centimeters per second, 600 centimeters/second to 800 centimeters/second, 600 centimeter s/second to 750 centimeters/second, 600 centimeters/second to 700 centimeters/second, 600 centimeter s/second to 650 centimeters/second, 650 centimeters/second to 900 centimeters per second, 650 centimeters/second to 850 centimeters per second, 650 centimeters/second to 800 centimeters/second, 650 centimeters/second to 750 centimeters/second, 650 centimeters/second to 700 centimeters/second, 700 centimeters/second to 900 centimeters per second, 700 centimeters/second to 850 centimeters per second, 700 centimeters/second to 800 centimeters/second, 700 centimeters/second to 750 centimeters/second, 750 centimeters/second to 900 centimeters per second, 750 centimeters/second to 850 centimeters per second, 750 centimeters/second to 800 centimeters/second, 800 centimeters/second to 900 centimeters per second, 800 centimeters/second to 850 centimeters per second, or 850 centimeters/second to 900 centimeters/second. As used herein, "impeller tip speed" refers to the speed of the outer most portion of the impeller as it rotates around its central axis.

In some embodiments, the agitating (and optionally additional steps as described herein) is performed in a container comprising an impeller, wherein a ratio of the impeller diameter to the container volume is 0.1 to 0.5, 0.1 to 0.4, 0.2 to 0.5, 0.2 to 0.4, 0.3 to 0.5, or 0.3 to 0.4.

In some embodiments, the agitating (and optionally additional steps as described herein) is performed in a container comprising an impeller, wherein a ratio of the impeller diameter to the inner diameter of the container is at least 0.25, at least 0.34, at least 0.65, 0.25 to 0.65, 0.25 to 0.33, 0.3 to 0.6, 0.3 to 0.5, 0.3 to 0.4, 0.34 to 0.65, 0.34 to 0.6, 0.34 to 0.55, 0.37 to 0.55, 0.4 to 0.65, 0.4 to 0.6, 0.4 to 0.5, or 0.42 to 0.55.

In some embodiments, agitating comprises mixing a cell composition or a lysed cell composition such that the cell composition or the lysed cell composition is placed under flow conditions described by a Reynolds number of 10 to 10,000, 1,000 to 10,000, 1,500 to 10,000, or 2,000 to 10,000. In some embodiments, a lysed cell emulsion during the agitating has a Reynolds number of 2,000 or more, 3,000 or more, or 5,000 or more, or 2,000 to 10,000, 3,000 to 10,000, or 5,000 to 10,000.

In some embodiments, a process comprises agitating a cell composition or a lysed cell composition for at least 5 minutes, at least 10 minutes, at least 20 minutes, at least 30 minutes, at least 1 hour, at least 2 hours, at least 4 hours, at least 8 hours, at least 12 hours, at least 18 hours, at least 24 hours, at least 30 hours, at least 36 hours, at least 42 hours, at least 48 hours, at least 54 hours, at least 60 hours, at least 66 hours, at least 72 hours, at least 78 hours, at least 84 hours, at least 90 hours or at least 96 hours. In some embodiments, a process comprises agitating a cell composition or a lysed cell composition for 5 minutes to 96 hours, 5 minutes to 4 hours, 5 minutes to 2 hours, 5 minutes to 1 hour, 10 minutes to 4 hours, 10 minutes to 2 hours, 10 minutes to 1 hour, 1 hour to 96 hours, 1 hour to 84 hours, 1 hour to 72 hours, 1 hour to 60 hours, 1 hour to 48 hours, 1 hour to 36 hours, 1 hour to 24 hours, 1 hour to 4 hours, 4 hours to 96 hours, 4 hours to 84 hours, 4 hours to 72 hours, 4 hours to 60 hours, 4 hours to 48 hours, 4 hours to 36 hours, 4 hours to 24 hours, 8 hours to 96 hours, 8 hours to 84 hours, 8 hours to 72 hours, 8 hours to 60 hours, 8 hours to 48 hours, 8 hours to 36 hours, 8 hours to 24 hours, 8 hours to 12 hours, 12 hours to 96 hours, 12 hours to 84 hours, 12 hours to 72 hours, 12 hours to 60 hours, 12 hours to 48 hours, 12 hours to 36 hours, 12 hours to 24 hours, 20 hours to 40 hours, 24 hours to 96 hours, 24 hours to 84 hours, 24 hours to 72 hours, 24 hours to 60 hours, 24 hours to 48 hours, or 24 hours to 36 hours.

In some embodiments, a process comprises simultaneously agitating and heating a concentrated cell composition or a lysed cell composition to demulsify the cell composition or the lysed cell composition. In some embodiments, a process comprises agitating a cell composition or a lysed cell composition at a temperature of at least 10°C, at least 20°C, at least 25°C, at least 30°C, at least 35°C, at least 40°C, at least 45°C, at least 50°C, at least 55°C, at least 60°C, at least 65°C, at least 70°C, at least 75°C, at least 80°C, at least 85°C, at least 90°C, at least 95°C, or at least 100°C. In some embodiments, a process comprises agitating a cell composition or a lysed cell composition at a temperature of 10°C to 100°C, 10°C to 90°C, 10°C to 80°C, 10°C to 70°C, 20°C to 100°C, 20°C to 90°C, 20°C to 80°C, 20°C to 70°C, 30°C to 100°C, 30°C to 90°C, 30°C to 80°C, 30°C to 70°C, 40°C to 100°C, 40°C to 90°C, 40°C to 80°C, 50°C to 100°C, 50°C to 90°C, 50°C to 80°C, 50°C to 70°C, 60°C to 100°C, 60°C to 90°C, 60°C to 80°C, 70°C to 100°C, 70°C to 90°C, 80°C to 1000°C, 80°C to 90°C, or 90°C to 100°C.

In some embodiments, the various combinations of forming a lysed cell composition, contacting a lysed cell composition with a first base or raising the pH of a lysed cell composition, contacting a lysed cell composition with a salt, heating the lysed cell composition, and agitating a lysed cell composition can occur in a single vessel. In some embodiments, the various combinations of forming a cell composition, contacting a cell composition with a base or raising the pH of a cell composition, contacting a cell composition with a salt, heating the cell composition, and agitating a cell composition can occur in a single vessel. In some embodiments, the single vessel includes a fermentation vessel. In some embodiments, the fermentation vessel can have a volume of at least 20,000 liters, at least 50,000 liters, at least 100,000 liters, at least 120,000 liters, at least 150,000 liters, at least 200,000 liters, or at least 220,000 liters. In some embodiments, the fermentation vessel can have a volume of 20,000 liters to 220,000 liters, 20,000 liters to 100,000 liters, 20,000 liters to 50,000 liters, 50,000 liters to 220,000 liters, 50,000 liters to 150,000 liters, 50,000 liters to 100,000 liters, 100,000 liters to 220,000 liters, 100,000 liters to 150,000 liters, 100,000 liters to 120,000 liters, 150,000 liters to 220,000 liters, 150,000 liters to 200,000 liters, or 200,000 liters to 220,000 liters.

In some embodiments, a quantity of cell composition or lysed cell composition formed in a vessel can be transferred into one or more agitation vessels. In some embodiments, the agitation vessels can have a volume of at least 20,000 liters, at least 30,000 liters, at least 40,000 liters or at least 50,000 liters, at least 100,000 liters, at least 150,000 liters, at least 200,000 liters, or higher. In some embodiments, the agitation vessels can have a volume of 20,000 liters to 50,000 liters, 20,000 liters to 40,000 liters, 20,000 liters to 30,000 liters, 30,000 liters to 50,000 liters, 30,000 liters to 40,000 liters or 40,000 liters to 50,000 liters, or higher.

In some embodiments, the agitation vessels can have any combination of the following properties. In some embodiments, the agitation vessels can have two impellers. In some embodiments, the impellers are Rushton blade impellers. In some embodiments, the impellers are separated from each other by a distance at least equal to a diameter of the smallest impeller. In some embodiments, the impellers are 76.2 cm (30 inches) to 101.6 cm (40 inches), 83.82 cm (33 inches) to 93.98 cm (37 inches), 86.36 cm (34 inches), 88.9 cm (35 inches), 91.44 cm (36 inches) or 93.98 cm (37 inches) from tip to tip. In some embodiments, the agitation vessels have a volume of at least 10,000 liters, at least 20,000 liters, at least 30,000 liters, at least 40,000 liters or at least 50,000 liters. In some embodiments, the agitation vessels have an inner diameter of 228.6 cm (90 inches) to 179.4 cm (110 inches), 241.3 cm (95 inches) to 266.7 cm (105 inches), 248.92 cm (98 inches), 251.46 cm (99 inches), 254 cm (100 inches), 256.54 cm (101 inches) or 259.08 cm (102 inches). In some embodiments, a first impeller is located 38.1 cm (15 inches) to 50.8 cm (20 inches), 40.64 cm (16 inches) to 48.26 cm (19 inches), or 43.18 cm (17 inches) to 45.72 cm (18 inches) from a bottom of the agitation vessel and a second impeller is located 152.4 cm (60 inches) to 203.2 cm (80 inches), 165.1 cm (65 inches) to 190.5 cm (75 inches), 172.72 cm (68 inches), 175.26 cm (69 inches), 177.8 cm (70 inches), 180.34 cm (71 inches), 182.88 cm (72 inches), 185.42 cm (73 inches), 187.96 cm (74 inches) or 190.5 cm (75 inches) above the first impeller. In some embodiment, a lysed cell composition is agitated at least 50 rpm, at least 60 rpm, or at least 70 rpm. In some embodiments, a lysed cell composition is agitated at 50 rpm to 70 rpm, 50 rpm to 60 rpm, 60 rpm to 70 rpm, 70 rpm to 100 rpm, 100 rpm to 150 rpm, 150 rpm to 200 rpm, 200 rpm to 250 rpm, or higher.

In some embodiments, the cell composition, the lysed cell composition, or the lipid are harvested from a vessel by pumping the cell composition, the lysed cell composition, or the lipid from the vessel. In some embodiments, the cell composition, the lysed cell composition, or the lipid are harvested from a vessel without agitating the vessel. In some embodiments, the cell composition, the lysed cell composition, or the lipid are harvested from a vessel by pumping, without agitation, the cell composition, the lysed cell composition, or the lipid from the vessel. In some embodiments, the cell composition, the lysed cell composition, or the lipid are harvested from a vessel without blowing air. In some embodiments, harvesting the cell composition, the lysed cell composition, or the lipid by the techniques described above, results in a crude lipid having a low anisidine value (e.g., 26 or less, 25 or less, 20 or less, 15 or less, 10 or less, 5 or less, 2 or less, or 1 or less) and/or a low phosphorus content (e.g., 100 ppm or less, 95 ppm or less, 90 ppm or less, 85 ppm or less, 80 ppm or less, 75 ppm or less, 70 ppm or less, 65 ppm or less, 60 ppm or less, 55 ppm or less, 50 ppm or less, 45 ppm or less, 40 ppm or less, 35 ppm or less, 30 ppm or less, 25 ppm or less, 20 ppm or less, 15 ppm or less, 10 ppm or less, 5 ppm or less, 4 ppm or less, 3 ppm or less, 2 ppm or less, or 1 ppm or less).

In some embodiments, the separating comprises centrifuging a treated cell composition or a treated lysed cell composition (e.g., at a temperature of 30°C to 100°C), whereby the centrifuging separates a lipid from the treated cell composition or the treated lysed cell composition.

In some embodiments, a process comprises centrifuging a treated cell composition or a treated lysed cell composition at a temperature of at least 10°C, at least 20°C, at least 25°C, at least 30°C, at least 35°C, at least 40°C, at least 45°C, at least 50°C, at least 55°C, at least 60°C, at least 65°C, at least 70°C, at least 75°C, at least 80°C, at least 85°C, at least 90°C, at least 95°C, or at least 100°C. In some embodiments, a process comprises centrifuging a treated cell composition or a treated lysed cell composition at a temperature of 10°C to 100°C, 10°C to 90°C, 10°C to 80°C, 20°C to 100°C, 20°C to 90°C, 20°C to 80°C, 25°C to 100°C, 25°C to 90°C, 25°C to 80°C, 25°C to 75°C, 30°C to 100°C, 30°C to 90°C, 30°C to 80°C, 40°C to 100°C, 40°C to 90°C, 40°C to 80°C, 50°C to 100°C, 50°C to 90°C, 50°C to 80°C, 50°C to 70°C, 60°C to 100°C, 60°C to 90°C, 60°C to 80°C, 60°C to 70°C, 70°C to 100°C, or 70°C to 90°C.

In some embodiments, centrifuging is conducted at a feed rate (of a treated cell composition or a treated lysed cell composition into a centrifuge) of 1 kilogram per minute (kg/min) to 500 kg/min, 1 kg/min to 400 kg/min, 1 kg/min to 300 kg/min, 1 kg/min to 200 kg/min, 1 kg/min to 100 kg/min, 1 kg/min to 75 kg/min, 1 kg/min to 50 kg/min, 1 kg/min to 40 kg/min, 1 kg/min to 30 kg/min, 1 kg/min to 25 kg/min, 1 kg/min to 10 kg/min, 10 kg/min to 500 kg/min, 10 kg/min to 400 kg/min, 10 kg/min to 300 kg/min, 10 kg/min to 200 kg/min, 10 kg/min to 100 kg/min, 10 kg/min to 75 kg/min, 10 kg/min to 50 kg/min, 10 kg/min to 40 kg/min, 10 kg/min to 30 kg/min, 20 kg/min to 500 kg/min, 20 kg/min to 400 kg/min, 20 kg/min to 300 kg/min, 20 kg/min to 200 kg/min, 20 kg/min to 100 kg/min, 20 kg/min to 75 kg/min, 20 kg/min to 50 kg/min, 20 kg/min to 40 kg/min, 25 kg/min to 500 kg/min, 25 kg/min to 400 kg/min, 25 kg/min to 300 kg/min, 25 kg/min to 200 kg/min, 25 kg/min to 100 kg/min, 25 kg/min to 75 kg/min, 25 kg/min to 50 kg/min, 30 kg/min to 60 kg/min, 30 kg/min to 50 kg/min, 30 kg/min to 40 kg/min, 50 kg/min to 500 kg/min, 100 kg/min to 500 kg/min, or 200 kg/min to 500 kg/min.

The total time required for the separating can vary depending on the volume of the treated cell composition or the treated lysed cell composition. Typical total time for separation (e.g., centrifuge time) is at least 30 seconds, at least 60 seconds, at least 2 minutes, at least 3 minutes, at least 4 minutes, at least 5 minutes, at least 0.1 hour, at least 0.2 hour, at least 0.5 hour, at least 1 hour, at least 2 hours, at least 4 hours, at least 6 hours, at least 8 hours, at least 10 hours, at least 12 hours, or 0.1 hour to 24 hours, 0.5 hour to 24 hours, 1 hour to 12 hours, 2 hours to 10 hours, or 4 hours to 8 hours.

In some embodiments, the process of the invention comprises centrifuging a treated cell composition or a treated lysed cell composition at a centrifugal force of 1.000 g to 25,000 g, 1,000 g to 20,000 g, 1,000 g to 10,000 g, 2,000 g to 25,000 g, 2,000 g to 20,000 g, 2,000 g to 15,000 g, 3,000 g to 25,000 g, 3,000 g to 20,000 g, 5,000 g to 25,000 g, 5,000 g to 20,000 g, 5,000 g to 15,000 g, 5,000 g to 10,000 g, 5,000 g to 8,000 g, 10,000 g to 25,000 g, 15,000 g to 25,000 g, or at least 1,000 g, at least 2,000, g, at least 4,000 g, at least 5,000 g, at least 7,000 g, at least 8,000 g, at least 10,000 g, at least 15,000 g, at least 20,000 g, or at least 25,000 g. As used herein, "g" refers to standard gravity or approximately 9.8 m/s². In some embodiments, a process of the invention comprises centrifuging a treated cell composition or a treated lysed cell composition at 4,000 rpm to 14,000 rpm, 4,000 rpm to 10,000 rpm, 6,000 rpm to 14,000 rpm, 6,000 rpm to 12,000 rpm, 8,000 to 14,000 rpm, 8,000 rpm to 12,000 rpm, or 8,000 rpm to 10,000 rpm.

In some embodiments, a process of the invention comprises drying a lipid after separation of the lipid from a treated cell composition or a treated lysed cell composition in order to remove water from the lipid. In some embodiments, drying the lipid can include, but is not limited to, heating the lipid to evaporate water. In some embodiments, after drying, the lipid has a water content by weight percentage of lipid that is less than 3%, less than 2.5%, less than 2%, less than 1.5%, less than 1%, less than 0.5%, less than 0.1%, or 0%. In some embodiments, after drying, the lipid has a water content by weight percentage of lipid of 0% to 3%, 0% to 2.5%, 0% to 2%, 0% to 1.5%, 0% to 1 %, 0% to 0.5%, 0.1% to 3%, 0.1% to 2.5%, 0.1% to 2%, 0.1% to 1.5%, 0.1% to 1%, 0.1% to 0.5%, 0.5% to 3%, 0.5% to 2.5%, 0.5% to 2%, 0.5% to 1.5%, 0.5% to 1%, 1% to 3%, 1% to 2.5%, 1% to 2%, 1% to 1.5%, 1.5% to 3%, 1.5% to 2.5%, 1.5% to 2%, 2% to 3%, 2% to 2.5%, or 2.5% to 3%.

In some embodiments, the process further comprises refining a lipid by one or more processes selected from caustic refining, degumming, alkali-refining, bleaching, deodorization, deacidification, or the like, and combinations thereof to remove one or more phospholipids, free fatty acids, phosphatides, color bodies, sterols, odors, and other impurities. As used herein, a "refined oil" is a crude lipid or crude oil that has been refined. In some embodiments, the processes of the current invention improve the yield of an RBD (refined, bleached, deodorized) or an RBDW (refined, bleached, deodorized and winterized) oil generated from the lipids extracted using the present invention. In various embodiments, the yield is improved at one or more of the refining, bleaching, deodorizing or winterizing steps in the refinement.

As used herein, "a crude lipid" or "a crude oil" is a lipid or oil that has not been refined. In some embodiments, the lipid separated from a demulsified cell composition is a crude lipid.

Various exemplary processes of the present invention are described schematically in FIG. 1.

In some embodiments, a broth comprising a microbial cell is concentrated to provide a lipid concentration of at least 4%, at least 5%, at least 10%, at least 15%, at least 20%, at least 25%, or at least 30% by weight of the broth. In some embodiments, a broth comprising a microbial cell is concentrated to provide a lipid concentration of 4% to 40%, 4% to 30%, 4% to 20%, 4% to 15%, 5% to 40%, 5% to 30%, 5% to 20%, 10% to 40%, 10% to 30%, 10% to 20%, 15% to 40%, 15% to 30%, 20% to 40%, 20% to 30%, 25% to 40%, or 30% to 40% by weight of the broth.

In some embodiments, a cell composition or a lysed cell composition is concentrated to provide a lipid concentration of at least 4%, at least 5%, at least 10%, at least 15%, at least 20%, at least 25%, at least 30%, at least 35%, at least 40%, at least 45%, at least 50%, at least 55%, or at least 60% by weight of the lysed cell composition. In some embodiments, a cell composition or a lysed cell composition is concentrated to provide a lipid concentration of 4% to 40%, 4% to 30%, 4% to 20%, 4% to 15%, 5% to 40%, 5% to 30%, 5% to 20%, 10% to 40%, 10% to 30%, 10% to 20%, 15% to 40%, 15% to 30%, 20% to 40%, 20% to 30%, 25% to 40%, or 30% to 40% by weight of the lysed cell composition.

In some embodiments of the present disclosure which are not according to the invention and are present for illustration purposes only, a lipid prepared by a process of the present invention has an overall aroma intensity of 2 or less. As used herein, the term "overall aroma intensity" refers to the olfactory sensory rating given to the lipid by a panel of sensory analysts. As used herein, the term "sensory analyst" refers to a trained individual that provides feedback on and/or rates the sensory characteristics of a substance.

In some embodiments of the present disclosure which are not according to the invention and are present for illustration purposes only, a lipid prepared by a process of the present invention has an overall aromatic intensity of 3 or less. As used herein, the term "overall aromatic intensity" refers to the gustatory, or taste, sensory rating given to the lipid by a panel of sensory analysts. In some embodiments, the Universal Spectrum descriptive analysis method is used to assess the aroma and aromatic characteristics of samples. This method uses an intensity scale of 0 - 15, where 0 = none detected and 15 = very high intensity, to measure the aroma and aromatic attributes of the oils.

In some embodiments of the present disclosure which are not according to the invention and are present for illustration purposes only, a lipid prepared by a process of the present invention does not have an aftertaste characterized as fishy. As used herein, the term "aftertaste" refers to the persistence of a sensation of a flavor in the lipid, as characterized by a panel of sensory analysts.

In some embodiments, the process of the present invention provides a crude lipid having a peroxide value (PV) of 5 or less, 4.5 or less, 4 or less, 3.5 or less, 3 or less, 2.5 or less, 2 or less, 1.5 or less, 1 or less, 0.5 or less, 0.2 or less, or 0.1 or less. As used herein, the terms "peroxide value" or "PV" refer to the measure of primary reaction products, such as peroxides and hydroperoxides, that occur during oxidation of the lipid. In some embodiments, the PV is an indicator of the quality of the lipid and the extent of oxidation which has occurred in the lipid having a low PV (i.e., 5 or less) demonstrates increased stability and sensory profiles than lipids having a PV greater than 5. In some embodiments, adding a base to a lysed cell composition, as discussed above, raises the pH of the lysed cell composition and inhibits lipid oxidation, thereby minimizing the number of free radicals in the lysed cell composition so that the crude lipid obtained from the processes of the invention has a low PV (i.e., 5 or less).

In some embodiments, the process of the present invention provides a crude lipid having an anisidine value (AV) of 26 or less, 25 or less, 20 or less, 15 or less, 10 or less, 5 or less, 2 or less, or 1 or less. As used herein, the terms "anisidine value" or "AV" refer to the measure of secondary reaction products, such as aldehydes and ketones, that occur during oxidation of the lipid. In some embodiments, the AV is an indicator of the quality of the lipid and the extent of oxidation which has occurred in the lipid. A lipid having a low AV (i.e., 26 or less) demonstrates increased stability and sensory profiles than lipids having an AV greater than 26. In some embodiments, adding a base to a lysed cell composition, as discussed above, raises the pH of the lysed cell composition and inhibits lipid oxidation, thereby minimizing the number of free radicals in the lysed cell composition so that the crude lipid obtained from the processes of the invention has a low AV (i.e., 26 or less).

In some embodiments, the process of the present invention provides a crude lipid having a phosphorus content of 100 ppm or less, 95 ppm or less, 90 ppm or less, 85 ppm or less, 80 ppm or less, 75 ppm or less, 70 ppm or less, 65 ppm or less, 60 ppm or less, 55 ppm or less, 50 ppm or less, 45 ppm or less, 40 ppm or less, 35 ppm or less, 30 ppm or less, 25 ppm or less, 20 ppm or less, 15 ppm or less, 10 ppm or less, 5 ppm or less, 4 ppm or less, 3 ppm or less, 2 ppm or less, or 1 ppm or less.

In some embodiments, the process of the present invention provides a crude lipid that has a lower anisidine value, lower peroxide value, lower phosphorus content and/or a higher extraction yield than if extraction was performed using a solvent (e.g., atypical hexane extraction or a FRIOLEX^{®} process (Westfalia Separator AG, Germany)). The FRIOLEX^{®} process which is a process of extracting lipids with a water-soluble organic solvent as described in U.S. Patent No. 5,928,696 and International Pub. Nos. WO 01/76385 and WO 01/76715.

In some embodiments, heating the lysed cell composition causes the secondary reaction products (e.g., aldehydes and ketones) to participate in a reaction similar to the Maillard reaction with proteins present in the lysed cell composition. The reaction is believed to create products that possess antioxidant activity, which reduces the oxidation of the lipid. In some embodiments, additional protein, e.g., soy protein, can be added to the lysed cell composition to increase the antioxidant activity. The reduction in oxidation of the lipid reduces the AV of the lipid, reduces any aftertaste of the lipid and/or increases the stability of the lipid. In some embodiments, the stability is increased at least 5%, at least 10%, at least 15% or at least 20%.

In some embodiments of the present disclosure which are not according to the invention and are present for illustration purposes only, a lipid extracted by a process of the present invention, the biomass remaining after extraction of the lipid, or combinations thereof can be used directly as a food or food ingredient, such as an ingredient in baby food, infant formula, beverages, sauces, dairy based foods (such as milk, yogurt, cheese and ice-cream), oils (e.g., cooking oils or salad dressings), and baked goods; nutritional supplements (e.g., in capsule or tablet forms); feed or feed supplement for any non-human animal (e.g., those whose products (e.g., meat, milk, or eggs) are consumed by humans); food supplements; and pharmaceuticals (in direct or adjunct therapy application); and in biofuel. The term "animal" refers to any organism belonging to the kingdom Animalia and includes any human animal, and non-human animal from which products (e.g., milk, eggs, poultry meat, beef, pork or lamb) are derived. In some embodiments, the lipid and/or biomass can be used in seafood. Seafood is derived from, without limitation, fish, shrimp and shellfish. The term "products" includes any product derived from such animals, including, without limitation, meat, eggs, milk or other products. When the lipid and/or biomass are fed to such animals, polyunsaturated lipids can be incorporated into the flesh, milk, eggs or other products of such animals to increase their content of these lipids.
Lipid Compositions - The following embodiments are not according to the invention and are present for illustration purposes only

In some embodiments, the present invention is directed to a microbial lipid extracted according to the processes of the present invention. In some embodiments, the microbial lipid has an anisidine value of 26 or less, 25 or less, 20 or less, 15 or less, 10 or less, 5 or less, 2 or less, or 1 or less, and/or a peroxide value of 5 or less, 4.5 or less, 4 or less, 3.5 or less, 3 or less, 2.5 or less, 2 or less, 1.5 or less, 1 or less, 0.5 or less, 0.2 or less, or 0.1 or less, and/or a phosphorus content of 100 ppm or less, 95 ppm or less, 90 ppm or less, 85 ppm or less, 80 ppm or less, 75 ppm or less, 70 ppm or less, 65 ppm or less, 60 ppm or less, 55 ppm or less, 50 ppm or less, 45 ppm or less, 40 ppm or less, 35 ppm or less, 30 ppm or less, 25 ppm or less, 20 ppm or less, 15 ppm or less, 10 ppm or less, 5 ppm or less, 4 ppm or less, 3 ppm or less, 2 ppm or less, or 1 ppm or less. In some embodiments, the lipid has less than 5%, less than 4%, less than 3%, less than 2%, or less than 1% by weight or volume of an organic solvent. In some embodiments, the lipid has at least 10%, at least 15%, at least 20%, at least 25%, at least 30%, at least 35%, at least 40%, at least 45%, or at least 50% by weight of a desired PUFA. In some embodiments, the lipid has at least 10%, at least 15%, at least 20%, at least 25%, at least 30%, at least 35%, at least 40%, at least 45%, or at least 50% by weight of DHA, and/or at least 10%, at least 15%, or at least 20% by weight of DPA n-6, and/or at least 10%, at least 15%, or at least 20% by weight of EPA, and/or at least 10%, at least 15%, at least 20%, at least 25%, at least 30%, at least 35%, at least 40%, at least 45%, or at least 50% by weight of ARA. In some embodiments the lipid extracted according to the processes of the present invention result in a lower anisidine value, lower peroxide value, lower phosphorus content and/or a higher extraction yield than if extraction was performed using a solvent (e.g., a typical hexane extraction or a FRIOLEX^{®} process (Westfalia Separator AG, Germany)).

A microbial lipid of the invention can be any lipid derived from a microorganism, including, for example: a crude oil extracted from the biomass of the microorganism without further processing; a refined oil that is obtained by treating a crude microbial oil with further processing steps such as refining, bleaching, and/or deodorizing; a diluted microbial oil obtained by diluting a crude or refined microbial oil; or an enriched oil that is obtained, for example, by treating a crude or refined microbial oil with further methods of purification to increase the concentration of a fatty acid (such as DHA) in the oil.

In some embodiments, the microbial lipid comprises a sterol esters fraction of 0%, at least 0.1%, at least 0.2%, at least 0.5%, at least about 1%, at least 1.5%, at least 2%, or at least 5% by weight. In some embodiments, the microbial lipid comprises a sterol esters fraction of from 0% to 1.5%, 0% to 2%, 0% to 5%, 1% to 1.5%, 0.2% to 1.5%, 0.2% to 2%, or 0.2% to 5% by weight. In some embodiments, the microbial lipid comprises a sterol esters fraction of less than 5%, less than 4%, less than 3%, or less than 2% by weight.

In some embodiments, the microbial lipid comprises a triglyceride fraction of at least 65%, at least 70%, at least 75%, at least 80%, at least 85%, or at least 90% by weight. In some embodiments, the microbial lipid comprises a triglyceride fraction of from 65% to 95%, 75% to 95%, or 80% to 95% by weight, or 97% by weight, or 98% by weight.

In some embodiments, the microbial lipid comprises a free fatty acid (FFA) fraction of at least 0.5%, at least 1%, at least 1.5%, at least 2%, at least 2.5%, or at least 5% by weight. In some embodiments, the microbial lipid comprises a free fatty acid fraction of from 0.5% to 5%, 0.5% to 2.5%, 0.5% to 2%, 0.5% to 1.5%, 0.5% to 1%, 1% to 2.5%, 1% to 5%, 1.5% to 2.5%, 2% to 2.5%, or 2% to 5% by weight. In some embodiments, the microbial lipid comprises a free fatty acid fraction of less than 5%, less than 4%, less than 3%, less than 2%, or less than 1% by weight.

In some embodiments, the microbial lipid comprises a sterol fraction of at least 0.5%, at least 1%, at least 1.5%, at least 2%, or at least 5% by weight. In some embodiments, the microbial lipid comprises a sterol fraction of from 0.5% to 1.5%, 1% to 1.5%, 0.5% to 2%, 0.5% to 5%, 1% to 2%, or 1% to 5% by weight. In some embodiments, the microbial lipid comprises a sterol fraction of less than 5%, less than 4%, less than 3%, less than 2%, or less than 1% by weight.

In some embodiments, the microbial lipid comprises a diglyceride fraction of at least 1.5%, at least 2%, at least 2.5%, at least 3%, at least 3.5%, or at least 5% by weight. In some embodiments, the microbial lipid comprises a diglyceride fraction of from 1.5% to 3%, 2% to 3%, 1.5% to 3.5%, 1.5% to 5%, 2.5% to 3%, 2.5% to 3.5%, or 2.5% to 5% by weight.

In some embodiments, the microbial lipid comprises unsaponifiables of less than 2%, less than 1.5%, less than 1%, or less than 0.5% by weight of the oil.

The lipid classes present in the microbial oil, such as the triglyceride fraction, can be separated by flash chromatography and analyzed by thin layer chromatography (TLC), or separated and analyzed by other methods know in the art.

In some embodiments, the microbial lipid and/or one or more fractions thereof selected from the triglyceride fraction, the free fatty acid fraction, the sterol fraction, the diglyceride fraction, and combinations thereof, comprises at least 40%, at least 45%, at least 50%, at least 55%, at least 60%, at least 65%, at least 70%, at least 75%, or at least 80% by weight DHA. In some embodiments, the microbial lipid and/or one or more fractions thereof selected from the triglyceride fraction, the free fatty acid fraction, the sterol fraction, the diglyceride fraction, and combinations thereof, comprises from 40% to 45%, 40% to 50%, 40% to 60%, 50% to 60%, 55% to 60%, 40% to 65%, 50% to 65%, 55% to 65%, 40% to 70%, 40% to 80%, 50% to 80%, 55% to 80%, 60% to 80%, or 70% to 80% by weight DHA. In some embodiments, the microbial lipid comprises a sterol esters fraction comprising 45% or less, 40% or less, 35% or less, 30% or less, 25% or less, 20% or less, 15% or less, or 13% or less by weight DHA. In some embodiments, the microbial lipid and/or one or more fractions thereof selected from the triglyceride fraction, the free fatty acid fraction, the sterol fraction, the diglyceride fraction, and combinations thereof, comprises 10% or less, 9% or less, 8% or less, 7% or less, 6% or less, 5% or less, 4% or less, 3% or less, 2% or less, or 1% or less by weight EPA. In some embodiments, the microbial lipid and/or one or more fractions thereof selected from the triglyceride fraction, the free fatty acid fraction, the sterol fraction, the diglyceride fraction, and combinations thereof, comprises from 2% to 3%, 2% to 3.5%, 2.5% to 3.5%, 2% to 6%, 2.5% to 6%, 3.0% to 6%, 3.5% to 6%, 5% to 6%, or 2% to 10% by weight EPA. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, is substantially free of EPA. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises a weight ratio of DHA to EPA of at least 5: 1, at least 7:1, at least 9: 1, at least 10: 1, at least 15:1, at least 20:1, at least 25:1, at least 30: 1, or at least 50: 1, wherein the microbial lipid and/or one or more fractions thereof comprises 10% or less by weight of EPA. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises a weight ratio of DHA to EPA of at least 5:1, but less than 20: 1. In some embodiments, the weight ratio of DHA to EPA is from 5:1 to 18:1, from 7:1 to 16:1, or from 10:1 to 15:1. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof comprises from 0.1% to 0.25%, 0.2% to 0.25%, 0.1% to 0.5%, or 0.1% to 1.5% by weight ARA. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises 1.5% or less, 1% or less, 0.5% or less, 0.2% or less, or 0.1% or less by weight ARA. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, is substantially free of ARA. In some embodiments, the microbial lipid and/or one or more fractions thereof selected from the sterol esters fraction, the triglyceride fraction, the free fatty acid fraction, the diglyceride fraction, the polar fraction (including the phospholipid fraction), and combinations thereof, comprises a weight ratio of DHA to ARA of at least 20:1, at least 30:1, at least 35: 1, at least 40: 1, at least 60: 1, at least 80: 1, at least 100: 1, at least 150:1, at least 200:1 , at least 250: 1 , or at least 300: 1. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises from 0.5% to 1%, 0.5% to 2%, 0.5% to 2.5%, 0.5% to 3%, 0.5% to 3.5%, 0.5% to 5%, 0.5% to 6%, 1% to 2%, 2% to 3%, 2% to 3.5%, 1% to 2.5%, 1% to 3%, 1% to 3.5%, 1% to 5%, or 1% to 6% by weight DP A n-6. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises 6% or less, 5% or less, 3% or less, 2.5% or less, 2% or less, 1% or less, or 0.5% or less by weight DPA n-6. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, is substantially free of DPA n-6. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises a weight ratio of DHA to DPA n-6 of greater than 6: 1, of at least 8:1, at least 10: 1, at least 15: 1, at least 20: 1, at least 25:1, at least 50: 1, or at least 100: 1. In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises 5% or less, 4% or less, 3% or less, 2% or less, 1.5% or less, 1% or less, or 0.5% or less by weight each of linoleic acid (18:2 n-6), linolenic acid (18:3 n-3), eicosaenoic acid (20:1 n-9), and erucic acid (22:1 n-9). In some embodiments, the microbial lipid and/or one or more fractions thereof selected from the sterol esters fraction, the triglyceride fraction, the free fatty acid fraction, the sterol fraction, the diglyceride fraction, the polar fraction (including the phospholipid fraction), and combinations thereof, comprises 5% or less, 4% or less, 3% or less, 2% or less, 1.5% or less, or 1% or less by weight of heptadecanoic acid (17:0). In some embodiments, the microbial lipid and/or one or more fractions thereof comprise 0.01% to 5% by weight, 0.05% to 3% by weight, or 0.1% to 1% by weight of heptadecanoic acid.

In some embodiments, an extracted microbial lipid comprises a triglyceride fraction of at least 70% by weight, wherein the docosahexaenoic acid content of the triglyceride fraction is at least 50% by weight, wherein the docosapentaenoic acid n-6 content of the triglyceride fraction is from at least 0.5% by weight to 6% by weight, and wherein the oil has an anisidine value of 26 or less. In some embodiments, an extracted microbial lipid comprises a triglyceride fraction of at least 70% by weight, wherein the docosahexaenoic acid content of the triglyceride fraction is at least 40% by weight, wherein the docosapentaenoic acid n-6 content of the triglyceride fraction is from at least 0.5% by weight to 6% by weight, wherein the ratio of docosahexaenoic acid to docosapentaenoic acid n-6 is greater than 6:1, and wherein the lipid has an anisidine value of 26 or less. In some embodiments, an extracted microbial lipid comprises a triglyceride fraction of at least 70% by weight, wherein the docosahexaenoic acid content of the triglyceride fraction is at least 60% by weight and wherein the lipid has an anisidine value of 26 or less. In some embodiments, an extracted microbial lipid having any of the above fatty acid profiles has an anisidine value of 26 or less, 25 or less, 20 or less, 15 or less, 10 or less, 5 or less, 2 or less, or 1 or less and/or a peroxide value of 5 or less, 4.5 or less, 4 or less, 3.5 or less, 3 or less, 2.5 or less, 2 or less, 1.5 or less, 1 or less, 0.5 or less, 0.2 or less, or 0.1 or less, and/or a phosphorus content of 100 ppm or less, 95 ppm or less, 90 ppm or less, 85 ppm or less, 80 ppm or less, 75 ppm or less, 70 ppm or less, 65 ppm or less, 60 ppm or less, 55 ppm or less, 50 ppm or less, 45 ppm or less, 40 ppm or less, 35 ppm or less, 30 ppm or less, 25 ppm or less, 20 ppm or less, 15 ppm or less, 10 ppm or less, 5 ppm or less, 4 ppm or less, 3 ppm or less, 2 ppm or less, or 1 ppm or less. In some embodiments, an extracted microbial lipid having any of the above fatty acid profiles is extracted from an isolated thraustochytrid microorganism having the characteristics of the thraustochytrid species deposited under ATCC Accession No. PTA-9695, PTA-9696, PTA-9697, or PTA-9698. In some embodiments, an extracted microbial lipid having any of the above fatty acid profiles is a crude lipid. In some embodiments, the crude lipid has less than 5% by weight or volume of an organic solvent. In some embodiments the microbial lipid extracted according to the processes of the present invention result in a lower anisidine value, lower peroxide value, lower phosphorus content and/or a higher extraction yield if extraction was performed using a solvent (e.g., atypical hexane extraction or a FRIOLEX^{®} process (Westfalia Separator AG, Germany)).

Having generally described the invention, a further understanding can be obtained by reference to the examples provided herein. These examples are given for purposes of illustration only and are not intended to be limiting. The following examples are illustrative, but not limiting, of a process and a lipid prepared by a process of the present invention. Other suitable modifications and adaptations of the variety of conditions and parameters normally encountered in extraction of a lipid from a cell, and which would become apparent to those skilled in the art.

### EXPERIMENTAL EXAMPLES

### Example 1 (not according to the invention). Lab-scale Biomass Concentration

Three 445 g samples from a pasteurized fermentation broth containing Schizochytrium microbial cells were heated to 90°C and pH adjusted to 10.2 with 50% Sodium hydroxide in three 0.5 L glass reactors. The temperature and pH treatment continued for 1.5 hours and then the temperature was decreased to 60°C and pH adjusted to 8.0 with 25% sulfuric acid solution. Sodium chloride (2.9 g) was added to the third reactor to determine whether separation was improved. Fifteen ml samples from each reactor and 15 ml samples from untreated broth (control) were centrifuged at 8000 rpm for 3 minutes and biomass separation compared. The result showed that there was poor separation for the control and the other three experiments had about 36% by volume of biomass plus aqueous phase clearly separated on the top of the broth.

### Example 2 (not according to the invention). Pilot trial Biomass Concentration and Lab Demulsification

1536 kg of pasteurized fermentation broth containing Schizochytrium was heated to 9°C and pH adjusted to 10.5 with 50% sodium hydroxide solution. After 1 hour, the pH drifted to 8.4 and was subsequently readjusted to 9.0. After an additional 1 hour, the temperature was decreased to 8°C and pH adjusted to 8.4 with 75% phosphoric acid. The treated broth was centrifuged at ~7000 x g and the broth was separated into three phases: the light phase consisting mainly of the biomass (primarily microbial cells, with some fermentation medium), the heavy phase consisting of mainly fermentation media (water, salts, sugar, and some residual microbial cells), and a third phase with similar composition to the heavy phase. A quantity of 146.7 kg of the light phase (concentrated biomass) with 33.4% moisture, 1381 kg heavy phase with 88.6% moisture, and 5.01 kg of a third phase with 84.64 kg moisture, was collected from the centrifuge. Samples from the light phase were demulsified with enzyme and without enzyme.

### Experiment A (lysis with enzyme)

A quantity of 33 8.7 g of concentrated biomass (light phase) from the pilot trial was heated to 66°C and the pH was adjusted from 8.3 to 7.55 by 50% citric acid solution under agitation. 37 mg Alcalase^{®} 2.4 FG (available from Novozymes (Franklinton, N.C.)) was added and lysis continued for 2 hours. At the end of two hours, the temperature was increased to 73°C and 10.1 g sodium chloride was added. Heating continued and, at 81°C, the pH was increased to 9.49. When the temperature reached 90°C, the pH drifted to 8.5 and was re-adjusted to 10.3. After 0.5 hour the pH was adjusted to 7.4 with 50% citric acid solution. Centrifugation of 15 ml showed complete demulsification and the reaction mass was collected in 50 ml centrifuge vials and centrifuged at 8000 rpm for 2.5 minutes. From the 338.7 g starting light phase, 203.2 g oil was collected.

### Experiment B (chemical lysis)

338.4 gm concentrated biomass (light phase) from the pilot trial was heated to 91°C and the pH was adjusted from 8.0 to 10.5 by 50% sodium hydroxide solution. After 12 minutes, 10.1 g sodium chloride was added. After 1 hour and 15 minutes, the pH was adjusted to 8.5 with 50% citric acid. 15 ml centrifugation showed complete demulsification and the reaction mass was collected in 50 ml vials and centrifuged at 8000 rpm for 2.5 minutes. From the 338.7 g starting light phase, 198.7 g oil was collected. A summary of the crude oil quality data and oil yield from

Experiment A and Experiment B is shown in Tables 1 and 2.

**Table 1: Crude oil quality data**

| | Experiment A | Experiment B |
|---|---|---|
| % Free Fatty Acid | 1.13 | 0.42 |
| Peroxide Value (meq/kg) | 0.47 | 0.71 |
| Anisidine Value (meq/kg) | 2.8 | 4.1 |
| % moisture | 0.58 | 0.57 |

**Table 2: Oil % yield data**

| Method of demulsification | Start wt. | % recovered in Light Phase | % lost in Heavy Phase | % lost in Third Phase | Free Fatty Acid (%) |
|---|---|---|---|---|---|
| Concentrated biomass-Ex. 1 | 146.7 kg | 92.3 | 13.4 | 0.4 | 0.56 |
| Concentrated biomass-Ex. 2, Exp. A (enzyme lysis) | 338.7 g | 94.3 | 5.9 | NA | 1.1 |
| Concentrated biomass-Ex. 2, Exp. B (chemical lysis) | 338.4 g | 91.0 | 9.7 | NA | 0.4 |

### Example 3. Pilot trial Biomass Concentration followed by pH Shock

2200.4 kg pasteurized fermentation broth containing Schizochytrium microbial cells was heated to 60°C and pH adjusted to 10.3 with 50% Sodium hydroxide. Once the temperature reached 86.5°C, the pH drifted to 8.7 and was adjusted to 10.2 by the addition of 50% sodium hydroxide solution under agitation. The temperature reached 90°C and this treatment condition continued for 1.5 hr. The pH was at 8.8. These temperature and pH treatments were performed to disrupt the surface chemistry of the cells in order to facilitate subsequent concentration of the biomass. The centrifugation was at ~7000 x g. The centrifugation separated the treated fermentation broth into the light phase consisting mainly of the biomass (primarily microbial cells, with some fermentation media), the heavy phase consisting of mainly fermentation media and some residual microbial cells, and the third phase consisting of a similar composition to the heavy phase. 191.2 kg concentrated biomass with 31.5 % moisture, 1995.6 kg heavy phase with 9.5 % moisture, and 16.5 kg third phase with 16.2 % moisture, was recovered from the three-phase centrifuge. The light phase was collected in a drum and left at room temperature overnight. The next day, the content in the drum was transferred to two 100 L tanks and the heating temperature set to 90°C. When the temperature reached 77°C, the pH was adjusted to 10.7 with 50% sodium hydroxide and heating continued until the temperature reached 90°C. At 90°C, the pH drifted to 9.9 and was readjusted to 10.7. After 2 hours of treatment at 90°C, the temperature was reduced to 82°C and the pH adjusted to 5.1 with 50% citric acid. After 30 minutes at this condition, the pH was adjusted to 10.6. This pH shock cycle was repeated 2 more times and the pH adjusted to 8.0. Sodium chloride (1.8 kg) was added to each tank and mixed for 30 minutes. 15 ml sampled from each tank was centrifuged and completeness of demulsification confirmed. The demulsified broth was centrifuged and separated into light phase (consisting mainly of crude oil) and heavy phase (consisting mainly of fermentation medium and aqueous material). A quantity of 117 kg of crude oil (light phase), 65.9 kg of heavy phase, and 7.73 kg of the third phase was collected from the centrifuged sample.

### Example 4 (Not according to the invention). Pilot trial (temperature shock included for lysis and demulsification)

2247.8 kg pasteurized fermentation broth with 86.1% moisture containing Schizochytrium strain of microalgae cells was heated to 80°C in 3000 L agitated tank. Once the temperature reached 80°C, the pH was adjusted to 10.7 by the addition of 50% sodium hydroxide solution under agitation. The broth was then heated for 2 hours. At the end of 2 hours, the pH drifted to 9.5. The pH was adjusted to 8.7 by adding 50% citric acid. These temperature and pH treatments were intended to disrupt the surface chemistry of the cells to facilitate subsequent concentration of the biomass. The treated broth was centrifuged at ~7000 x *g*. The centrifugation separated the treated fermentation broth into the light phase consisting mainly of the biomass with some fermentation media, heavy phase consisting of mainly fermentation media and some residual microbial cells, and the third phase, consisting of a composition similar to the heavy phase. 187.4 kg light phase with 37.1% moisture, 2051 kg heavy phase with 89.6 % moisture and 11.7 kg of the third phase with 84.5% moisture, were collected from the centrifuge output. The concentrated biomass in the light phase was collected in a 750 L tank where it was cooled to 10°C overnight.

On the second day, 2215 kg pasteurized fermentation broth from the same batch as above was heated to 70°C and pH was adjusted to 10.7. Heating continued to 80°C and pH adjusted to 10.7 again. After 2 hours of treatment, the pH was adjusted to 8.7 with a 50% solution of citric acid. The treated broth was centrifuged. At approximately half way through the centrifugation step, the pH was adjusted to 7.6 while centrifugation was ongoing. 240.5 kg light phase with 46.7 % moisture, 1999.3 kg heavy phase with 89.9% moisture and 8.9 kg of the third phase with 85.7 % moisture, were collected from the centrifuge output. The concentrated biomass in the light phase was collected in the same 750 L tank above where it was cooled to 10°C overnight. Enough concentrated biomass was collected for lysis and demulsification in the 750 L tank. The concentrated biomass from two batches was heated to 80°C in the 750 L tank under agitation. The pH was at 8.3. The pH was adjusted to 7.8 with 50% citric acid under agitation. A 15 ml sample was evaluated, and centrifugation showed complete demulsification. However, 4 kg NaCl salt was added and mixed for 30 minutes. The pH was dropped to 5.9 with 50% citric acid. After mixing for 30 minutes, the pH raised to 8.2. A 15 ml sample centrifuged on bench centrifuge showed complete demulsification.

The demulsified composition was centrifuged on a pilot centrifuge. A quantity of 225.6 kg crude oil with 3.16% moisture (light phase), 182.6 kg heavy phase with 87.06% moisture, and 2.76 kg of the third phase with 82.5% moisture, was collected from the centrifuged material.

**Table 3: Recovery Data for Biomass Concentration Pilot Trial**

| | AEX-O-1216 Day 1 | AEX-O-1216 Day 2 | Mix of Light Phase-Day 1 and Day 2-demulsification |
|---|---|---|---|
| Starting weight (kg) | 2246.8 | 2213.8 | 434.63 |
| Total Start DHA +EPA +DPA n-3 (kg) | 87.03 | 78.98 | 141.51 |
| Weight-Light Phase (Concentrated Biomass)-(kg) | 186.4 | 240.5 | 227.73 (crude oil) |
| Total DHA+EPA+DPA n-3 Light Phase (kg) | 68.48 | 71.28 | 130.76 |
| Weight-Heavy Phase (kg) | 2051 | 1999.3 | 182.6 |
| Total DHA+EPA+DPA n-3 Heavy Phase (kg) | 15.45 | 10.79 | 1.32 |
| Weight-Third Phase (kg) | 11.68 | 8.94 | 2.76 |
| Total DHA+EPA+DPA n-3 Third Phase (kg) | 0.31 | 0.14 | 0.08 |
| % Product recovered in Light Phase | 78.7 | 90.2 | 92.4 |
| % Product recovered in Heavy Phase | 17.8 | 13.7 | 0.9 |
| % Product lost in Third Phase | 0.4 | 0.2 | 0.1 |

## Claims

1. A process for obtaining a lipid from a composition comprising microbial cells, the process comprising:
(a) treating the composition comprising the microbial cells by heating the composition to a temperature of 60°C to 95°C, preferably from 80°C to 95°C, at a pH of 9 to 11 under agitation for a period of time from 1 hour to 6 hours;
(b) separating the treated microbial cells into a light phase consisting essentially of a concentrated microbial cell composition and a heavy phase consisting essentially of fermentation medium and aqueous material;
(c) adjusting the pH of the concentrated microbial cell composition to a pH of 10 to 12 by adding a base and maintaining the pH of 10 to 12 for at least 1 hour;
(d) adjusting the pH of the composition of (c) to a pH of 3 to 6 by adding an acid and maintaining the pH of 3 to 6 for at least 0.5 hours;
(e) optionally repeating steps (c) and (d) until the composition is sufficiently demulsified;
(f) separating the lipid from the demulsified lysed cell composition; and
(g) recovering the lipid.

2. The process of claim 1, wherein (c) and (d) comprise heating the composition to 70°C to 90°C.

3. The process of claim 1 or claim 2, wherein, prior to (c), one or more enzymes capable of disrupting the cell wall of the microbial cells is added to the concentrated microbial cell composition for a time sufficient for lysis of the microbial cells, preferably wherein the enzyme is selected from betaglucanase, xylanase, cellulase, protease, pectinase, mannanase, amylase, and combinations thereof.

4. The process of any preceding claim, wherein the composition is subjected to one of more cycles of temperature shock before (c), after (c), before (d), after (d), or a combination thereof, preferably wherein the temperature shock comprises adjusting the temperature from 80-90°C to 4-20°C and back to 80-90°C.

5. The process of any preceding claim, wherein (b), (c), and/or (d) further comprises adding a salt in an amount of from 0.05% to 20%, by weight, of the concentrated microbial cell composition, wherein the salt is selected from the group consisting of alkali metal salts, alkali earth metal salts, sulfate salts, and combinations thereof.

6. The process of any preceding claim, wherein (a) further comprises mechanical disruption of the cells wherein the mechanical disruption results in lysis of less than 5% of the cells.

7. The process of any preceding claim, wherein the cells of (a) are unwashed and/or are contained in a fermentation broth.

8. The process of any preceding claim, wherein (f) comprises centrifugation or membrane filtration of the demulsified lysed cell composition.

9. The process of any preceding claim, wherein
(i) the pH is adjusted to 7 .5-8.5 prior to (b) and/or
(ii) the temperature is adjusted to 70-80°C prior to (b).

10. The process of any preceding claim, wherein the lipid comprises a polyunsaturated fatty acid, wherein the polyunsaturated fatty acid is selected from an omega-3 fatty acid, an omega-6 fatty acid, and mixtures thereof, preferably wherein the polyunsaturated fatty acid is selected from docosahexaenoic acid (DHA), eicosapentaenoic acid (EPA), docosapentaenoic acid (DPA), arachidonic acid (ARA), gamma-linolenic acid (GLA), di-homo-gamma-linolenic acid (DGLA), stearidonic acid (SDA), and mixtures thereof, more preferably, wherein the polyunsaturated fatty acid is docosahexaenoic acid (DHA), eicosapentaenoic acid (EPA) or arachidonic acid (ARA).

11. The process of any preceding claim, wherein the microbial cells are one or more of algae, yeast, fungi, protist, or bacteria cells, preferably wherein the microbial cells are from the genus Mortierella, genus Crypthecodinium, or order Thraustochytriales, more preferably, wherein the microbial cells are from the genus Thraustochytrium, Schizochytrium, or mixtures thereof, or from Mortierella alpina.

12. The process of any preceding claim, wherein the lysed cell composition comprises liquid, cell debris, and microbial oil and/or wherein an organic solvent is not used to obtain the lipid from the cells.

13. The process of any preceding claim, wherein the lipid of (g) is a crude lipid, preferably wherein (g) further comprises refining the crude lipid to obtain a refined lipid.

14. The process of any preceding claim, wherein the lipid
(iii) comprises at least 30% by weight docosahexaenoic acid;
(iv) comprises at least 20% by weight eicosapentaenoic acid (EPA);
(v) comprises at least 30% by weight arachidonic acid;
(vi) has an anisidine value of less than 26;
(vii) has a phosphorus content of 100 ppm or less; and/or
(viii) has a peroxide value of less than 5 meq/kg.

## Patentansprüche

1. Verfahren zum Erhalt eines Lipids aus einer Zusammensetzung, die mikrobielle Zellen umfasst, wobei das Verfahren:
(a) das Behandeln der Zusammensetzung, die die mikrobiellen Zellen umfasst, durch Erwärmen der Zusammensetzung auf eine Temperatur von 60 °C bis 95 °C, vorzugsweise von 80 °C bis 95 °C, bei einem pH von 9 bis 11 unter Rühren für einen Zeitraum von 1 Stunde bis 6 Stunden;
(b) das Trennen der behandelten mikrobiellen Zellen in eine leichte Phase, die hauptsächlich aus einer konzentrierten mikrobiellen Zellzusammensetzung besteht, und eine schwere Phase, die hauptsächlich aus Fermentationsmedium und wässerigem Material besteht;
(c) das Einstellen des pHs der konzentrierten mikrobiellen Zellzusammensetzung auf einen pH von 10 bis 12 durch Zugeben einer Base und Beibehalten des pHs von 10 bis 12 für mindestens 1 Stunde;
(d) das Einstellen des pHs der Zusammensetzung von (c) auf einen pH von 3 bis 6 durch Zugeben einer Säure und Beibehalten des pHs von 3 bis 6 für mindestens 0,5 Stunden;
(e) gegebenenfalls das Wiederholen der Schritte (c) und (d), bis die Zusammensetzung ausreichend demulgiert ist;
(f) das Abtrennen des Lipids aus der demulgierten lysierten Zellzusammensetzung; und
(g) das Gewinnen des Lipids
umfasst.

2. Verfahren nach Anspruch 1, wobei (c) und (d) das Erwärmen der Zusammensetzung auf 70 °C bis 90 °C umfassen.

3. Verfahren nach Anspruch 1 oder Anspruch 2, wobei vor (c) der konzentrierten mikrobiellen Zellzusammensetzung für eine Zeit, die für Lyse der mikrobielle Zellen ausreicht, ein oder mehrere Enzym(e) zugegeben wird/werden, das/die die Zellwand der mikrobielle Zellen aufbrechen kann/können, wobei das Enzym vorzugsweise aus Beta-Glucanase, Xylanase, Cellulase, Protease, Pectinase, Mannanase, Amylase und Kombinationen davon ausgewählt ist.

4. Verfahren nach einem vorstehenden Anspruch, wobei die Zusammensetzung vor (c), nach (c), vor (d), nach (d) oder einer Kombination davon einem von mehreren Temperaturschockzyklen unterzogen wird, wobei der Temperaturschock vorzugsweise das Einstellen der Temperatur von 80 - 90 °C auf 4 - 20 °C und zurück auf 80 - 90 °C umfasst.

5. Verfahren nach einem vorstehenden Anspruch, wobei (b), (c) und/oder (d) ferner das Zugeben eines Salzes in einer Menge von 0,05 % bis 20 %, bezogen auf das Gewicht, der konzentrierten mikrobiellen Zellzusammensetzung umfassen/umfasst, wobei das Salz aus der Gruppe ausgewählt ist, die aus Alkalimetallsalzen, Erdalkalimetallsalzen, Sulfatsalzen und Kombinationen davon besteht.

6. Verfahren nach einem vorstehenden Anspruch, wobei (a) ferner das mechanische Aufbrechen der Zellen umfasst, wobei das mechanische Aufbrechen zu Lyse von weniger als 5 % der Zellen führt.

7. Verfahren nach einem vorstehenden Anspruch, wobei die Zellen von (a) ungewaschen und/oder in einer Fermentationsbrühe enthalten sind.

8. Verfahren nach einem vorstehenden Anspruch, wobei (f) die Zentrifugation oder Membranfiltration der demulgierten lysierten Zellzusammensetzung umfasst.

9. Verfahren nach einem vorstehenden Anspruch, wobei
(i) der pH vor (b) auf 7,5 - 8,5 eingestellt wird und/oder
(ii) die Temperatur vor (b) auf 70 - 80 °C eingestellt wird.

10. Verfahren nach einem vorstehenden Anspruch, wobei das Lipid eine mehrfach ungesättigte Fettsäure umfasst, wobei die mehrfach ungesättigte Fettsäure aus einer Omega-3-Fetetsäure, einer Omega-6-Fettsäure und Gemischen davon ausgewählt ist, wobei die mehrfach ungesättigte Fettsäure vorzugsweise aus Docosahexaensäure (DHA), Eicosapentaensäure (EPA), Docosapentaensäure (DPA), Arachidonsäure (ARA), Gamma-Linolensäure (GLA), Di-homo-gamma-linolensäure (DGLA), Stearidonsäure (SDA) und Gemischen davon ausgewählt ist, wobei die mehrfach ungesättigte Fettsäure Docosahexaensäure (DHA), Eicosapentaensäure (EPA) oder Arachidonsäure (ARA) ist.

11. Verfahren nach einem vorstehenden Anspruch, wobei die mikrobiellen Zellen eine oder mehrere von Algen-, Hefe-, Pilz-, Einzeller- oder Bakterienzellen sind, wobei die mikrobiellen Zellen vorzugsweise aus der Gattung Mortierella, der Gattung Crypthecodinium oder der Ordnung Thraustochytriales stammen, wobei die mikrobiellen Zellen stärker bevorzugt aus der Gattung Thraustochytrium, Schizochytrium oder Gemischen davon oder Mortierella alpina stammen.

12. Verfahren nach einem vorstehenden Anspruch, wobei die lysierte Zellzusammensetzung Flüssigkeit, Zelltrümmer und mikrobielles Öl umfasst und/oder wobei kein organisches Lösungsmittel zum Erhalt des Lipids aus den Zellen verwendet wird.

13. Verfahren nach einem vorstehenden Anspruch, wobei das Lipid von (g) ein Rohlipid ist, wobei (g) vorzugsweise ferner das Aufreinigen des Rohlipids unter Erhalt eines aufgereinigten Lipids umfasst.

14. Verfahren nach einem vorstehenden Anspruch, wobei das Lipid
(iii) mindestens 30 Gew.-% Docosahexaensäure umfasst;
(iv) mindestens 20 Gew.-% Eicosapentaensäure (EPA) umfasst;
(v) mindestens 30 Gew.-% Arachidonsäure umfasst;
(vi) eine Anisidinzahl von weniger als 26 aufweist;
(vii) einen Phosphorgehalt von 100 ppm oder weniger aufweist; und/oder
(viii) eine Peroxidzahl von weniger als 5 meq/kg aufweist.

## Revendications

1. Procédé pour obtenir un lipide à partir d'une composition comprenant des cellules microbiennes, le procédé comprenant les étapes consistant à :
(a) traiter la composition comprenant les cellules microbiennes par chauffage de la composition à une température de 60 °C à 95 °C, de préférence de 80 °C à 95 °C, à un pH de 9 à 11 sous agitation pendant une période de temps de 1 heure à 6 heures ;
(b) séparer les cellules microbiennes traitées en une phase légère consistant essentiellement en une composition concentrée de cellules microbiennes et une phase lourde consistant essentiellement en un milieu de fermentation et une matière aqueuse ;
(c) ajuster le pH de la composition concentrée de cellules microbiennes à un pH de 10 à 12 en ajoutant une base et maintenir le pH de 10 à 12 pendant au moins 1 heure ;
(d) ajuster le pH de la composition de (c) à un pH de 3 à 6 en ajoutant un acide et maintenir le pH de 3 à 6 pendant au moins 0,5 heure ;
(e) répéter éventuellement les étapes (c) et (d) jusqu'à ce que la composition soit suffisamment désémulsifiée ;
(f) séparer le lipide de la composition de cellules lysées désémulsifiées ; et
(g) récupérer le lipide.

2. Procédé selon la revendication 1, dans lequel les étapes (c) et (d) comprennent le chauffage de la composition de 70°C à 90°C.

3. Procédé selon la revendication 1 ou la revendication 2, dans lequel, avant (c), une ou plusieurs enzymes capables de perturber la paroi cellulaire des cellules microbiennes sont ajoutées à la composition de cellules microbiennes concentrée pendant un temps suffisant pour lyser les cellules microbiennes, de préférence dans lequel l'enzyme est choisie parmi la bêta-glucanase, la xylanase, la cellulase, la protéase, la pectinase, la mannanase, l'amylase et leurs combinaisons.

4. Procédé selon l'une quelconque des revendications précédentes, dans lequel la composition est soumise à un ou plusieurs cycles de choc thermique avant (c), après (c), avant (d), après (d), ou une combinaison de ceux-ci, de préférence dans lequel le choc thermique comprend l'ajustement de la température de 80 à 90 °C à 4 à 20 °C, puis de nouveau à 80 à 90 °C.

5. Procédé selon l'une quelconque des revendications précédentes, dans lequel (b), (c) et/ou (d) comprennent en outre l'ajout d'un sel en une quantité comprise entre 0,05 % et 20 % en poids de la composition de cellules microbiennes concentrée, dans lequel le sel est choisi parmi le groupe constitué des sels de métaux alcalins, des sels de métaux alcalino-terreux, des sels de sulfate et de leurs combinaisons.

6. Procédé selon l'une quelconque des revendications précédentes, dans lequel (a) comprend en outre la rupture mécanique des cellules, dans lequel la rupture mécanique entraîne la lyse de moins de 5 % des cellules.

7. Procédé selon l'une quelconque des revendications précédentes, dans lequel les cellules de (a) ne sont pas lavées et/ou sont contenues dans un bouillon de fermentation.

8. Procédé selon l'une quelconque des revendications précédentes, dans lequel (f) comprend la centrifugation ou la filtration sur membrane de la composition de cellules lysées désémulsifiées.

9. Procédé selon l'une quelconque des revendications précédentes, dans lequel
(i) le pH est ajusté à 7,5-8,5 avant (b) et/ou
(ii) la température est ajustée à 70-80 °C avant (b).

10. Procédé selon l'une quelconque des revendications précédentes, dans lequel le lipide comprend un acide gras polyinsaturé, dans lequel l'acide gras polyinsaturé est choisi parmi un acide gras oméga-3, un acide gras oméga-6 et leurs mélanges, de préférence dans lequel l'acide gras polyinsaturé est choisi parmi l'acide docosahexaénoïque (DHA), l'acide eicosapentaénoïque (EPA), acide docosapentaénoïque (DPA), l'acide arachidonique (ARA), l'acide gamma-linolénique (GLA), l'acide di-homo-gamma-linolénique (DGLA), l'acide stéaridonique (SDA) et leurs mélanges, de préférence, dans lequel l'acide gras polyinsaturé est l'acide docosahexaénoïque (DHA), l'acide eicosapentaénoïque (EPA) ou l'acide arachidonique (ARA).

11. Procédé selon l'une quelconque des revendications précédentes, dans lequel les cellules microbiennes sont une ou plusieurs cellules d'algues, de levures, de champignons, de protistes ou de bactéries, de préférence dans lequel les cellules microbiennes sont du genre Mortierella, du genre Crypthecodinium ou de l'ordre Thraustochytriales, de préférence dans lequel les cellules microbiennes sont du genre Thraustochytrium, Schizochytrium ou des mélanges de ceux-ci, ou de Mortierella alpina.

12. Procédé selon l'une quelconque des revendications précédentes, dans lequel la composition de cellules lysées comprend un liquide, des débris cellulaires et une huile microbienne et/ou dans lequel un solvant organique n'est pas utilisé pour obtenir le lipide à partir des cellules.

13. Procédé selon l'une quelconque des revendications précédentes, dans lequel le lipide de (g) est un lipide brut, de préférence dans lequel (g) comprend en outre le raffinage du lipide brut pour obtenir un lipide raffiné.

14. Procédé selon l'une quelconque des revendications précédentes, dans lequel le lipide
(iii) comprend au moins 30 % en poids d'acide docosahexaénoïque ;
(iv) comprend au moins 20 % en poids d'acide eicosapentaénoïque (EPA) ;
(v) comprend au moins 30 % en poids d'acide arachidonique ;
(vi) a une valeur d'anisidine inférieure à 26 ;
(vii) a une teneur en phosphore de 100 ppm ou moins ; et/ou
(viii) a une valeur de peroxyde inférieure à 5 meq/kg.
